(19) Europäisches Patentamt
European Patent Office
Office européen des brevets

(11) Veröffentlichungsnummer: **0 000 742**
**B1**

(12) **EUROPÄISCHE PATENTSCHRIFT**

(45) Veröffentlichungstag der Patentschrift: **12.05.82**

(51) Int. Cl.³: **C 07 J 5/00**, C 07 J 31/00, A 61 K 31/57

(21) Anmeldenummer: **78100524.4**

(22) Anmeldetag: **27.07.78**

(54) **Corticoid-17-Alkylcarbonate und Verfahren zu deren Herstellung und diese enthaltende Mittel.**

(30) Priorität: **04.08.77 DE 2735110**

(43) Veröffentlichungstag der Anmeldung:
**21.02.79 Patentblatt 79/4**

(45) Bekanntmachung des Hinweises auf die Patenterteilung:
**12.05.82 Patentblatt 82/19**

(84) Benannte Vertragsstaaten:
**BE CH DE FR GB NL SE**

(56) Entgegenhaltungen:
**LU - A - 68 212**
**US - A - 3 558 675**

(73) Patentinhaber: **HOECHST AKTIENGESELLSCHAFT**
**Postfach 80 03 20**
**D-6230 Frankfurt/Main 80 (DE)**

(72) Erfinder: **Stache, Ulrich, Dr.**
**Goldgraben 20**
**D-6238 Hofheim am Taunus (DE)**
Erfinder: **Fritsch, Werner, Dr.**
**Fuchshohl 1**
**D-6232 Bad Soden am Taunus (DE)**
Erfinder: **Alpermann, Hans Georg, Dr.**
**Am Eichkopf 10**
**D-6240 Königstein/Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 742 B1

Corticoid-17-Alkylcarbonate und Verfahren zu deren Herstellung und diese enthaltende Mittel

Gegenstand der Erfindung sind neue Steroid-17-alkylcarbonate der Formel I oder II

I

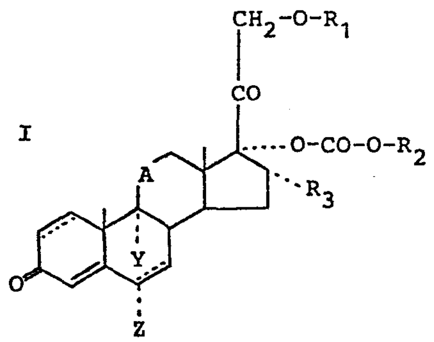

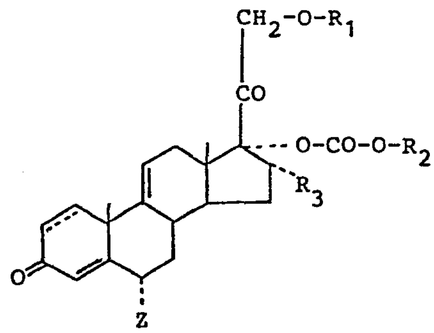

II

in welcher bedeuten:

A $=CHOH$ in beliebiger sterischer Anordnung $=CH_2$ oder $=CO$

Y Wasserstoff, Fluor oder Chlor,

Z Wasserstoff, Fluor, Chlor oder Methyl

$R_3$ Wasserstoff, Fluor, $\alpha$-Methyl, Monofluormethyl oder Difluormethyl,

$R_2$ Alkyl mit 1—8 C-Atomen

$R_1$ Acyl der formel III

$$-CO-(CH_2)_n-R_4 \qquad III$$

Carbonylalkyl der Formel IV

$$-CO-O-(CH_2)_n-R_4 \qquad IV$$

oder Sulfoalkyl der Formel V

$$-SO_2-R_5 \qquad V$$

in welcher bedeutet

$R_4$ Wasserstoff, Alkyl mit 1—10 C-Atomen, Cycloalkyl mit 3—6 C-Atomen oder, wenn n 0, Fluor, Chlor, Brom, Piperidyl

$R_5$ Alkyl mit 1—4 C-Atomen, Phenyl, Methylphenyl, Äthylphenyl, Fluorphenyl, Bromphenyl oder Chlorphenyl

n eine ganze Zahl zwischen 0 und 4.

Gegenstand der Erfindung ist ferner ein Verfahren zur Herstellung dieser Verbindungen, welches dadurch gekennzeichnet ist, daß man Verbindungen der Formeln VI oder VII

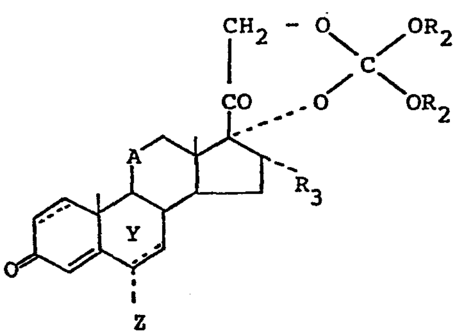

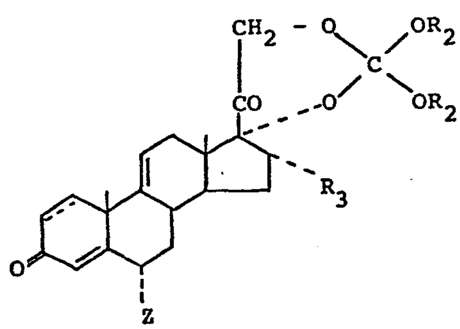

mit einer schwachen Säure hydrolysiert und die erhaltene 21-Hydroxy-Verbindung mit einem Halogenid oder Anhydrid einer Carbonsäure der Formel VIII

$$R_4-(CH_2)_n-CO-OH \qquad VIII$$

einem Halogenformat der Formel IX

$$R_4-(CH_2)_n-O-CO\text{-Halogen} \qquad IX$$

oder einen Sulfonsäurehalogenid der Formel X

$$R_5\text{—}SO_2\text{-Halogen} \qquad X$$

verestert.

Von den für die Reste $R_2$, $R_4$ und $R_5$ genannten Bedeutungen sind die folgenden bevorzugt:

Für $R_2$: Alkyl mit 1—5 C-Atomen,

Für $R_5$: Methyl, Äthyl, Propyl, Phenyl und die anderen für $R_5$ genannten substituierten Phenylreste, wobei die Substituenten jeweils in p-Stellung stehen.

Besonders bevorzugt sind Verbindungen der Formel I, worin Y und Z nicht Halogen bedeuten.

Die als Ausgangsubstanz benötigten Steroid-17 21-Dialkylorthocarbonate der Formeln VI und VII sind bekannt und können z.B. gemäß der DP—S 16 68 079 hergestellt werden. Als Ausgangsstoffe kommen insbesondere die 17 21-Dialkylorthocarbonate folgender 17 21-Dihydroxysteroide bzw. -corticoide in Betracht:

Cortison, Hydroxycortison, Reichsteins Substanz S, Prednison, Prednisolon, 6α-Methylprednisolin, 16α- oder 16β-Methylprednisolon, 9α-Fluor- oder 9α-Chlor-prednisolon, 16-Methylenprednisolon, 6α, 9α-Difluorprednisolon, 6α-Methyl-9α-prednisolon, 6α-Fluor-prednisolon, 9α-Fluor-16α-methyl-prednisolon, 9α - Fluor - prednisolon, 9α - Fluor - 16α - methyl - prednisolon, 9α-Fluor-prednisolon, 9α - Fluor - 16 - methyl - prednisolon, 6α - Fluor - 16α - methyl - prednisolon, 6α - Fluor - 16β - methyl - prednisolon, 6α - Fluor - 16 - methylen - prednisolon, 6α,9α - Difluor - 16α - methyl - prenisolon, 6α,9α - Difluor - 16β - methyl - prednisolon, 6α,9α - Difluor - 16 - methylen - prednisolon, 9α - Fluor - 6α,16α - dimethylprednisolon, 9α,16α-Difluor-prednisolon, 6α,9α-Trifluor-prednisolon, 17α,21 - Dihydroxy - $\Delta^{4(5),9(11)}$ - pregnadien - dion - (3, 20), 17α,21 - Dihydroxy - 9β - 11β - oxido - $\Delta^4$ - pregnen - dion - (3,20), 17α,21 - Dihydroxy - 9α,11β - dichlor - $\Delta^{1,4}$ - pregnadien - dion - (3, 20), 17α,21 - Dihydroxy - $\Delta^{4(5),6(7)}$ - pregnadien - dion - (3, 20), Desoxycorticosteron, Corticosteron, 16α-Methyl-corticosteron, 9α - Fluor - 16α - methyl - corticosteron, 6α,9α - Difluor - 16α - methyl - corticosteron, 6α - Fluor - 16α - methyl - corticosteron, 6,16α - 2 - Dimethyl - 4,6 - pregnadien - 11β - 17α,21 - triol - [3,2-c] - 2′ - phenylpyrazol bzw. -2′-p-fluorphenylpyrazol bzw. deren in 9α-Position durch Fluor substituierten Analoge. Ferner kommen solche der genannten Corticoide in Betracht, die anstelle einer 6α-Fluor- und/oder 9α-Fluor- und/oder 11β-Hydroxygruppe ein in entsprechender Konfiguration orientiertes Chloratom aufweisen.

In der ersten Reaktionsstufe des Verfahrens, nämlich der protonenkatalysierten Hydrolyse des Steroid-17 ,21-dialkylorthocarbonates zu einer entsprechenden Steroid - 17 - monoalkylcarbonat - 21 - hydroxy - Verbindung wird vorzugsweise eine Carbonsäure wie beispielsweise Ameisen-, Essig-, Propion-, Butter-, Valerian-, Oxal-, Malein-, Fumar-, Bernstein- oder Adipinsäure, oder eine *organische Sulfonsäure,* wie beispielsweise p-Toluol-, Benzol-, p- oder o- oder m-Chlor- oder -Brom-benzolsulfonsäure oder eine *anorganische Säure,* wie beispielsweise Salz-, Schwefel-, Kohlen-, Salpetersäure, verwendet. Dabei ist der Reaktionsverlauf zu den gewünschten Steroid - 17,- monoalkyl - carbonat - -21 - hydroxy - Verbindungen umso spezifischer je schwächer die Säure ist, d.h. je näher man im pH-Wert an den Wert 7 heran kommt. Das ist umso überraschender, als beide am Kohlenstoffatom der Orthokohlensäuregruppierung verknüpften Alkoxy-Liganden gleichwertig sind, also nicht, wie beispielsweise bei formal ähnlich strukturierten 17 ,21-Steroid-carbonsäureorthoestern ungleichwertig sind, und auf diese Weise keine den letzteren Liganden zukommenden Regiospezifität hinsichtlich einer bevorzugten Orthoesteraufspaltung entwickeln bzw. induzieren können. Um einen gewünschten pH-Wert mit den genannten Säuren einzustellen, ist es oft zweckmäßig, zum Verdünnen Wasser oder/und inerte organische Lösungsmittel, wie beispielsweise Alkohole, lineare oder cyclische Äther, Ester, Dialkylformamide, Dialkylsulfoxyde oder Hexamethylphosphorsäuretriamid zuzugeben, wobei neben dem Verdünnungseffekt oft ein katalytischer oder regioselektiver effekt in Richtung des gewünschten Reaktionsverlaufs bewirkt wird.

Der Reaktionsablauf in der gewünschten Richtung wird zweckmäßigerweise durch Dünnschichtchromatographie verfolgt. Es ist vorteilhaft, die Reaktion durch Neutralisieren beispielsweise mit verdünntem Ammoniak oder Einstellen auf pH-Wert von über 7 abzubrechen, wenn das Dünnschichtdiagramm nach optimaler Bildung der gewünschten Steroid-17 -monoalkylcarbonat-21-hydroxy-Verbindungen auf deren Isometrisierung zu den nicht gewünschten Steroid-17 -hydroxy-21-monoalkylcarbonat-Verbindungen hinweisen.

Vorzugsweise löst man das Corticoid-17,21-orthoalkylcarbonat in einer Carbonsäure, wie beispielsweise in Essigsäure oder Propionsäure, setzt vorzugsweise etwa 0,1 bis 1% Wasser zu und läßt das Gemisch bis zu ca. 8 Stunden bei einer Temperatur von 0° bis zum Siedepunkt der verwendeten Säure oder Lösungsmittel reagieren. Wenn im DC-Diagramm eine optimale Bildung des gewünschten Produkts festgestellt worden ist, rührt man das Reaktionsgemisch in Wasser oder Kochsalzlösung ein, neutralisiert beispielsweise mit wässrigem Ammoniak oder einer anderen schwachen Base, saugt entweder den Niederschlag ab oder extrahiert in üblicher Weise mit organischen Lösungsmitteln, dampft ein, kristallisiert die erhaltenen Produkte um und chromatographiert diese, falls im DC-Diagramm noch Ausgangsmaterial oder bereits 21-Alkylcarbonat nachweisbar ist, erforderlichenfalls am Kieselgel oder Aluminiumoxid.

Die 21-Hydroxygruppe kann, je nachdem ob ein 21-Alkylcarbonat, ein 21-Carbonsäurederivat oder ein 21-Alkyl- oder -Arylsulfonsäureester der zugrundeliegenden Corticoid-17-alkylcarbonate hergestellt werden soll, mit den dazu üblichen Acylierungsmitteln umgesetzt werden:

a) Zur Herstellung von 21-Alkylcarbonaten werden vorzugsweise Chlorameisensäurealkylester der Formel

$$Cl—\underset{\underset{\displaystyle O}{\|}}{C}—O—(CH_2)_n—R_4$$

verwendet, in der $R_4$ die zur Formel I angegebene Bedeutung hat. Vorzugsweise wird Chlorameisensäure-methylester, -äthylester, -propylester oder -butylester verwendet.

b) Zur Herstellung von 21-Carbonsäureestern werden vorzugsweise entweder Carbonsäurehalogenide der Formel

$$Hal—\underset{\underset{\displaystyle O}{\|}}{C}—(CH_2)_n—R_4,$$

in der Hal Cl, Br oder J darstellt und $R_4$ die zur Formel I angegebene Bedeutung hat, oder Carbonsäureanhydride der Formel $(OC—(CH_2)_n—R_4)_2O$, in der $R_4$ die zur Formel I angegebene Bedeutung hat, verwendet. Beispielsweise können verwendet werden: Essigsäure-, Propionsäure, Buttersäure-, Valeriansäure-chlorid oder -anhydrid, Cyclopropancarbonsäure-, Cyclopentylpropionsäure- oder Önanthsäurechlorid.

c) Zur Herstellung von 21-Sulfonsäureestern kommen Sulfonsäurehalogenide der Formel $Cl—SO_2—R_5$, in der $R_5$ die zur Formel I angegebene Bedeutung hat, in Frage. Vorzugsweise werden Methansulfonsäure- oder o-, m- oder p-Toluolsulfonsäurechlorid eingesetzt.

Für die zweite Verfahrensstufe löst man die Steroidkomponente in einem inerten Lösungsmittel, wie beispielsweise in einem Äther, wie Dioxan, Tetrahydrofuran, Diglym, oder gegebenenfalls halogenierten Kohlenwasserstoff, wie Benzol, Toluol, Cyclohexan, Methylenchlorid, Chloroform oder in einem Gemisch dieser Lösungmittel. Zur Entfernung der in der Reaktion entstehenden Halogenwasserstoffsäure setzt man 1—1000 Moläquivalente einer tertiären Base wie beispielsweise Pyridin, Chinolin, Triäthylamin oder Dimethylanilin zu. Man kann aber auch eine an organische Base wie Natriumhydrogencarbonat oder Calciumcarbonat zur Entfernung der Säure benutzen. Anschließend tropft man 1—200 Moläquivalente, vorzugsweise 1—3 Moläquivalente eines der oben angeführten Acylierungsmittel, gegebenenfalls gelöst in einem der oben angeführten Lösungsmittel, bei einer Temperatur zwischen —40°C und dem Siedepunkt des verwendeten Lösungsmittels vorzugsweise zwischen 0°C und 25°C, zu. Anschließend läßt man das Reaktionsgemisch eine bis 120 Stunden bei einer Temperatur zwischen —40°C und dem Siedepunkt des Lösungsmittels, vorzugsweise zwischen 0°C und 25°C stehen.

Bei Verwendung von Carbonsäureanhydriden als Acylierungsmittel ist es oft von Vorteil, ohne Zusatz von Lösungsmitteln zu arbeiten. Es reicht in der Regel aus, lediglich die organische Base, vorzugsweise Pyridin, dem imn Überschuß angewandten Säureanhydrid zuzufügen.

Zur Aufarbeitung gießt man das Reaktionsgemisch in Wasser, das gegebenenfalls mit Natriumbicarbonat versetzt wurde, wobei die Reaktionsprodukte, oft erst nach längerem Stehen, im allgemeinen kristallin ausfallen. Ölig gebliebene Reaktionsprodukte werden durch Ausschütteln mit einem geeigneten Extraktionsmittel und Eindampfen angereichert. Die Reaktionsprodukte können, falls erforderlich, durch Umkristallisieren oder durch Chromatographie aufgetrennt oder gereinigt werden. Oft genügt auch intensives Digerieren in einem das Reaktionsprodukt möglichst wenig oder nicht lösenden organischen Lösungsmittel, wie Diäthyläther oder Cyclohexan oder einem Gemisch aus diesen Komponenten, zur weiteren Reinigung der Reaktionsprodukte.

Eine Hydroxygruppe in 11-Stellung kann gegebenenfalls nach üblichen Methoden zur Ketogruppe oxydiert werden. Vorzugsweise wird diese Oxydation mit Chromtrioxid in saurem Medium und in einem inerten organischen Lösungsmittel durchgeführt.

Die Verfahrensprodukte besitzen wertvolle pharmakologische Eigenschaften. Sie sind insbesondere lokal und topisch sehr stark antiphlogistisch wirksam und zeigen teilweise ein vorteilhaftes Verhältnis von lokaler zu systemischer antiinflammtorischer Wirkung, wie aus pharmakologischen Standardtests hergeleitet werden kann.

Demgemäß ist Gegenstand der Erfindung auch ein Mittel zur Behandlung entzündlicher Dermatosen bestehend aus oder enthaltend eine Verbindung der Formel I oder II.

Die Verfahrensprodukte können in der Veterinär- und Humantherapie zur Behandlung von entzündlichen Dermatosen verschiedenster Genese in Form von Suspensionen, Salben, Cremes, Sprays usw. Verwendung finden. Bei topischer Anwendung können sie in Form von Kristallsuspensionen — z.B. bei intraartikulärer Injektion — appliziert werden. Dabei ist als besonders vorteilhaft für die lokale und topische Therapieform herauszuheben, daß die Verfahrensprodukte aufgrund ihres günstigen Verhältnisses von lokaler zu systemischer antiphlogistischer Wirkung auch bei hochdosierten un langanhaltender Therapie praktisch nur geringfügige systemische Nebenwirkungen hervorrufen können. Darüberhinaus weisen die eingesetzten Verfahrensprodukte eine signifikant bessere Säurestabilität auf als die ihnen zugrundeliegenden cyclischen Corticoid-17,21-ortho-carbonate. Dieser Tatbestand ist für eine sichere und therapiegereichte Anmeldung der erfindungsgemäßen Produkte von ausschlaggebender Bedeutung.

Bei äußerlicher Behandlung werden Salben, Cremes, Suspensionen usw. mit einer Konzentration von 0,01 bis 2 (Gew.-)%, bei topischen Verabreichungen in Form von lokalen (nicht-systemischen) Injektionen Dosierungen von 0,1 mg bis 100 mg verwendet.

Zu den im folgenden aufgeführten Beispielen sind die nachstehenden allgemeinen Bemerkungen zu machen:

Die Schmelzpunkte werden im Apparat nach Tottoli (Fa. Büchi) bestimmte und sind nicht korrigiert.

Die IR-Spektren (in KBr) werden mit dem Gitterspektrophotometer Perkin-Elmer 521 aufgenommen. Es werden jeweils nur die charakteristischen Banden angeführt. Die Aufnahme der UV-Spektren (in Methanol) erfolgte mit dem Spektralphotometer Beckman DK 1 A. Die massenspektroskopischen Untersuchungen (MS) werden mit dem Gerät MS 9 (Fa. AEI) durchgeführt.

Für die Dünnschichtchromatographie (DC) dienten Fertigplatten Kieselgel $F_{254}$ (Fa. Merck).

Wenn nich anders angegeben, wurde als Laufmittel Methylenchlorid : Methanol = 19 : 1 benutzt. Es wurde jeweils einmal entwickelt. Die Flecken wurden durch Besprühen mit 10%-iger methanolischer Schwefelsäure sowie durch Erhitzen auf 100°C sichtbar gemacht. Die $R_f$-Werte sind immer nur relativ zu verstehen. Zur Säulenchromatographie wurde Kieselgel 60, Korngröße 0,063—0,2 mm (Fa. Merck) verwendet.

Beispiel 1

a) Eine Lösung von 3 g Dexamethason-17,21-diäthylorthocarbonat in 120 ml Eisessig und 0.6 ml Wasser wird 5 Stunden bei 22°C stehen gelassen. Eine DC—Überprüfung ergab, daß nach dieser Zeit eine optimale Menge an dem gewünschten Dexamethason-17-äthylcarbonat vorhanden war.

Man gießt das Reaktionsgemisch in 1,5 l Wasser ein, das mit Ammoniak-Lösung auf pH=5 gebracht worden war, wobei ein kristalliner Niederschlag ausfiel. Nach dem Abfiltrieren Waschen mit Wasser und Trocknen erhählt man nach dem Digerieren 1,8 g Dexamethason-17-äthylcarbonat vom Schmp. 154° (Tottoli). Das zurückgebliebene wässrige Filtrat wird mit Methylenchlorid extrahiert. Nach dem Abdestillieren des Lösungsmittels bleibt ein schaumiger Rückstand zurück, der aus Diisopropyläther zur Kristallisation gebracht wird und weitere 1,2 g Dexamethason-17-äthylcarbonat von Schmp. 152° ergibt. Beide Präparationen 1,8 + 1,2 g werden vereinigt und aus Äthanol umkristallisiert.

Schmp. 156°C (Tottoli)

*Charakter. IR—Banden:* 3440, 2940, 2880, 1735, 1720, 1660, 1610, 1265 $cm^{-1}$

*Massenspektrum*: Mol-Gew. Peak bei $M^{\oplus}$: 464

*DC*: $R_f = 0{,}43$

$(CH_2Cl_2: CH_3OH = 19:1)$

b) Eine Lösung von 4 g Dexamethason-17,21-dimethylorthocarbonat in 250 ml Eisessig und 1 ml Wasser wird 15 Min. bei 20°C stehengelassen und danach in 2,5 l halbgesättigten Kochsalzlösung eingerührt. Nach analoger Aufarbeitung und Weiterbehandlung wie unter Beispiel 1) a) angegeben, erhält man 3,2 g Dexamethason-17-methylcarbonat.

*Massenspektrum*: $M^+$: 450

*DC*: $R_f = 0{,}42$

c) Eine Lösung von 4,5 g Dexamethason-17,21-di-(n-propyl)-orthocarbonat in 280 ml Eisessig und 1,2 ml Wasser wird 5 Stunden bei 20°C stehengelassen und auf 4 l halbgesättigte Kochsalzlösung gegossen. Man dekantiert von der öligen Ausfällung ab, nimmt das Öl in Methylenchlorid auf und wäscht mit Wasser. Nach dem Abdestillieren des Lösungsmittels erhält man 3,3 g Dexamethason-17-n-propylcarbonat als amorphen Schaum, der ohne Weiterbehandlung in die Folgereaktionen eingesetzt wird.

*Charakter. IR-Banden:* 3440, 1730, 1655, 1610, 1240 $cm^{-1}$

*Massenspektrum*: $M^+ = 476$

*DC*: $R_f = 0{,}42$

d) In gleicher Weise, wie in Beispiel 1) c) beschrieben, werden 4,5 g Dexamethason-17,21-di-(n-butyl)-orthocarbonat umgesetzt und aufgearbeitet. Man erhält nach dem Digerieren mit Diisopropyläther 2,9 g Dexamethason-17-n-butylcarbonat vom Schmp. 92°C.

*IR*: 3430, 1730, 1655, 1605, 1270 $cm^{-1}$

e) In gleicher Weise, wie in Beispiel 1) c) beschrieben, werden 4,5 g Dexamethason - 17,21 - di - (n - pentyl) - orthocarbonat (Fp. 106°), hergestellt aus Dexamethason und Tetra-n-pentylorthocarbonat gemäß DBP 1 668 079, umgesetzt und aufgearbeitet. Man erhält 3,4 g amorphes Dexamethason-17-n-pentylcarbonat.

*IR*: 3440, 1735, 1660, 1610, 1275 $cm^{-1}$

Beispiel 2

a) Zu einer Lösung von 1,4 g Dexamethason-17-äthylcarbonat in 9 ml abs. Dioxan und 4,5 ml Pyridin wird bei ca. 0°C eine Lösung von 1,1 g Chlorameisensäureäthylester in 9 ml Dioxan getropft. Nach 5 Stunden Rühren bei 0°C gießt man in ca. 300 ml halbgesättigte wässrige Kochsalzlösung ein, extrahiert mit Methylenchlorid, wäscht die organische Phase mit Wasser, dampft das Lösungsmittel im Vakuum ein und erhält 1,4 g Dexamethason-17,21-bis-[äthylcarbonat] vom Schmp. 202—204°C.

Im DC zeigt das Produkt neben dem starken Hauptfleck bei $R_f = 0,57$ noch schwache Nebenflecke bei $R_f = 0,47$ und 0,33 Zur Reinstdarstellung wird daher das Reaktionsprodukt an Kieselgel (Säule 3 x 10 cm) mit säurefreiem Methylenchlorid als Aufzieh- und Eluationsmittel chromatographisch aufgetrennt. Die Fraktionen, in denen aufgrund des DC-Diagramms ausschließlich das gewünschte Verfahrensprodukt identifiziert wird ($R_f = 0,57$), werden vereinigt und aus Äthanol/Äther kristallisiert.

Man erhält 1,2 g Dexamethason-17,21-bis-(äthylcarbonat) vom Schmp. 210°C

*DC*: $R_f = 0,57$ (keine Nebenflecke)

*IR*: 3420, 1735, 1660, 1610, 1260 $cm^{-1}$

b) In gleicher Weise, wie unter Beispiel 2 a) beschrieben, werden 1,4 g Dexamethason-17-äthylcarbonat mit 1,1 g Chlorameisensäure-methylesters statt des -äthylesters umgesetzt und aufgearbeitet.

Man erhält das Dexamethason-17-äthylcarbonat-21-methyl-carbonat

*IR*: 3420, 1740, 1665, 1615, 1260 $cm^{-1}$

c) In gleicher Weise, wie unter Beispiel 2 b) beschrieben, werden 1,4 g Dexamethason-17-äthylcarbonat mit 1,2 g Chlorameisensäure-propylester statt des -äthylesters umgesetzt und aufgearbeitet.

Man erhält das Dexamethason-17-äthylcarbonat-21-n-propyl-carbonat.

*IR*: 3420, 1735, 1660, 1615, 1265 $cm^{-1}$

d) In gleicher Weise, wie unter Beispiel 2 b) beschrieben, werden 1,4 g Dexamethason-17-äthylcarbonat mit 1,3 g Chlorameisen-säure-n-butylester statt des -äthylesters umgesetzt und aufgearbeitet.

Man erhält das Dexamethason-17-äthylcarbonat-21-n-butyl-carbonat.

*IR*: 3420, 1735, 1660, 1610, 1265 $cm^{-1}$

e) In gleicher Weise, wie unter Beispiel 2 b) beschrieben, werden 1,4 g Dexamethason-17-äthylcarbonat mit 1,2 g Chlorameisensäure-iso-propylester statt des -äthylesters umgesetzt und aufgearbeitet.

Man erhält das Dexamethason-17-äthylcarbonat-21-iso-propylcarbonat.

*IR*: 3420, 1735, 1665, 1615, 1265 $cm^{-1}$

f) Zu einer Lösung von 3 g Dexamethason-17-äthylcarbonat in 35 ml abs. Aceton und 12 ml abs. Pyridin werden bei 0°C 5 ml Methansulfonsäurechlorid zugetropft. Nach 20 Stunden Rühren bei 0°C bis 22°C (allmählich Temperatur ansteigen lassen) wird in Wasser eingegossen, mit Methylenchlorid extrahiert, gewaschen und das Extraktionsmittel im Vak. eingeengt. Der Rückstand wird an Kieselgel (Säule 4 x 14 cm) mit Methylenchlorid als Eluationsmittel chromatographiert. Die DC-reinen Fraktionen mit $R_f = 0,62$ werden vereinigt und aus Äthanol/Äther kristallisiert.

Man erhält 2,6 g Dexamethason-17-äthylcarbonat-21-methansulfonat vom Schmp. 193°C.

Massenspektrum: $M^+ = 542$

*IR*: 3430, 1730, 1655, 1610, 1600, 1350, 1265, 1170, 1030 $cm^{-1}$

g) Zu einer Lösung von 1 g Dexamethason-17-äthylcarbonat in 12 ml abs. Pyridin werden 0,3 ml Cyclopropancarbonsäurechlorid getropft. Nach 24 Stunden Rühren bei 20°C wird auf Wasser/NaCl-Lösung gegossen und der Niederschlag abfiltriert. Ausbeute 1 g. Nach der Chromatographie an Kieselgel (Säule 2 x 10 cm) mit Methylenchlorid gegebenenfalls unter Zusatz von 2% Methanol, werden die Fraktionen mit nur einem Fleck bei $R_f = 0,6$ vereinigt und aus Äthanol/Äther kristallisiert.

Man erhält 730 mg Dexamethason-17-äthylcarbonat-21-cyclo-propancarbonsäureester vom Schmp. 219°C

*IR*: 3440, 1730, 1660, 1610, 1260 $cm^{-1}$

MS-Spektrum: $M^+ = 532$

h) Zu einer Lösung von 1 g Dexamethason-17-äthylcarbonat in 12 ml Pyridin tropft man bei 0°C 0,26 ml Propionsäurechlorid und rührt anschließend 3 Stunden bei 20°C. Man gießt auf Wasser, neutralisiert mit verd. Salzsäure, trennt das ausgefallene Öl ab, nimmt es in Methylenchlorid auf, wäscht

mit Wasser, engt im Vakuum ein chromatographiert wie in Beispiel 2 g angegeben, und kristallisiert den Rückstand aus Äther um.

Man erhält 817 mg Dexamethason-17-äthylcarbonat-21-n-propionat vom Schmp. 220—222°C;
DC: $R_f = 0,6$
*IR*: 3450, 1730, 1660, 1610, 1600, 1260 $cm^{-1}$
MS-Spektrum: $M^+ = 520$

i) In gleicher Weise wie unter Beispiel 2 h) beschrieben werden 1 g Dexamethason-17-äthylcarbonat mit Acetylchlorid statt Propionsäurechlorid umgesetzt und aufgearbeitet.

Man erhält das Dexamethason-17-äthylcarbonat-21-acetat vom Schmp. 236—240°
*IR*: 3460, 1740, 1660, 1610, 1265 $cm^{-1}$

Zum gleichen Produkt gelangt man, wenn man statt Acetylchlorid 4 ml Acetanhydrid wählt und nach 16 Stunden Stehen bei 20° in analoger Weise aufarbeitet.

j) In gleicher Weise, wie unter Beispiel 2 h) beschrieben, werden 1 g Dexamethason-17-äthylcarbonat mit 0,3 ml Buttersäurechlorid statt Propionsäurechlorid umgesetzt und aufgearbeitet.

Man erhält das Dexamethason-17-äthylcarbonat-21-butyrat vom Schmp. 202—205°C.

k) In gleicher Weise, wie unter Beispiel 2 h) beschrieben, werden 1 g Dexamethason-17-äthylcarbonat mit 0,4 ml Valeriansäurechlorid statt Propionsäurechlorid umgesetzt und aufgearbeitet.

Man erhält das Dexamethason-17-äthylcarbonat-21-valerat.
*IR*: 3460, 1735, 1660, 1610, 1260 $cm^{-1}$

l) In gleicher Weise, wie unter Beispiel 2 g) beschrieben, werden 1 g Dexamethason-17-äthylcarbonat mit 1,28 g Cyclopentylpropionsäurechlorid, gelöst in 3 ml absol. Dioxan, statt des Cyclopropancarbonsäurechlorids umgesetzt und aufgearbeitet.

Man erhält 645 mg Dexamethason-17-äthylcarbonat-21-cyclopentyl-propionat vom Schmp. 202°C
*IR*: 3440, 1735, 1660, 1600, 1265 $cm^{-1}$
*MS-Spektrum*: $M^+ = 588$

## Beispiel 3

a) Zu einer Lösung von 1 g Dexamethason-17-methylcarbonat in 6 ml abs. Dioxan und 4 ml abs. Pyridin wird unter Rühren bei 0°C eine Lösung von 0,32 ml Chlorameisensäuremethylester in 2 ml abs. Dioxan getropft. Nach 5 Stunden Rühren bei Raumtemperatur wird in 300 ml halbgesättigte wässrige Kochsalzlösung gegossen, das ausgefallene Kristallisat abfiltriert, mit Wasser gewaschen und getrocknet. Man erhält 1,2 g rohes Dexamethason-17,21-bis-[methylcarbonat], das an Kieselgel (Säule 3 × 13 cm) mit Methylenchlorid chromatographiert wird. Die Fraktionen, die im DC ausschließlich eine Punkt bei $R_f = \sim 0,55$ zeigen, werden vereinigt und aus Alkohol/Äther kristallisiert.

Man erhält das Dexamethason-17,21-bis-(methylcarbonat) vom Schmp. 250°C
DC: $R_f = 0,55$
*IR*: 3460, 1740, 1655, 1610, 1440, 1275 $cm^{-1}$
MS-Spektrum: $M^+ = 508$

b) Zu einer Lösung von 700 mg DC-einheitlichem Dexamethason-17-methylcarbonat in 4,5 ml Dioxan und 2,8 ml Pyridin wird bei 0° und unter Rühren eine Lösung von 550 mg Chlorameisensäureäthylester in 2,7 ml Dioxan getropft. Nach weiteren 16 Stunden Rühren bie 0°C wird in wässrige Kochsalzlösung eingerührt, das ausgefallene Kristallisat abfiltriert, getrocknet (710 mg) und aus Aceton/Äther umkristallisiert. Man erhält 630 mg DC-einheitliches ($R_f = 0,53$) Dexamethason-17-methylcarbonat-21-äthylcarbonat vom Schmp. 249°
*IR*: 3460, 1740, 1655, 1610, 1440, 1265 $cm^{-1}$
Massenspektrum: $M^+ = 522$

c) In gleicher Weise, wie in Beispiel 3 b) beschrieben, werden 700 mg Dexamethason-17-methylcarbonat mit

1) 600 mg Chlorameisensäure-n-propylester,
2) 650 mg Chlorameisensäure-n-butylester,
3) 600 mg Chlorameisensäure-iso-propylester,
4) 650 mg Chlorameisensäure-iso-butylester statt des Chlorameisensäureäthylesters umgesetzt und aufgearbeitet. Man erhält so jeweils das entsprechende

1) Dexamethason-17-methylcarbonate-21-n-propylcarbonat,
2) -21-n-butylcarbonat,
3) -21-iso-propylcarbonat,
4) -21-iso-butylcarbonat

d) In gleicher Weise, wie in Beispiel 2 f) beschrieben, werden 3 g Dexamethason-17-methylcarbonat mit Methansulfonsäurechlorid umgesetzt und aufgearbeitet.

Man erhält 2,4 g Dexamethason-17-methylcarbonat-21-methansulfat vom Schmp. 210—214° (Zers.)

*MS*-Spektrum: $M^+ = 528$

e) Zu einer Lösung von 600 mg Dexamethason-17-methylcarbonat in 3 ml Pyridin und 5 ml Dioxan werden 0,5 ml Cyclopropancarbonsäurechlorid, in 1 ml Dioxan gelöst, bei 0°C und unter Rühren getropft. Nach 5 Stunden Rühren bei 20°C wird auf Wasser/Kochsalzlösung gegossen und der Niederschlag abfiltriert. Ausbeute 510 mg. Nach analoger chromatographie, wie in Beispiel 2 g) angegeben, erhählt man aus Aceton/Äther den Dexamethason-17-methylcarbonat-21-cyclopropancarbonsäureester vom Schmp. 274°C

*IR*: 3460, 1735, 1655, 1610, 1435, 1270 $cm^{-1}$

MS-Spektrum: $M^+ = 518$

f) Zu einer Lösung von 700 mg Dexamethason-17-methylcarbonat in 8,4 ml Pyridin tropft man bei 0° 0,2 ml Propionsäurechlorid in 1 ml Dioxan und rührt anschließend 3 Stunden bei 22°C. Man gießt auf Wasser, neutralisiert mit verd. Salzsäure, filtriert den Niederschlag ab, wäscht ihn mit Wasser und trocknet ihn. Bei einem weiteren Ansatz, bei dem das Reaktionsprodukt ölig ausgefallen ist, trennt man das Öl ab, nimmt es mit Methylenchlorid auf, wäscht mit Wasser und engt im Vakuum ein. Das jeweils erhaltene Reaktionsprodukt wird, wie in Beispiel 2 g angegeben, chromatographiert. Nach dem Umkristallisieren aus Aceton/Äther erhält man 550 mg Dexamethason-17-methylcarbonat-21-n-propionat vom Schmp. 260°C

*IR*: 3460, 1735, 1655, 1610, 1435, 1270 $cm^{-1}$

MS-Spektrum: $M^+ = 506$

g) In gleicher Weise, wie in Beispiel 3 f) beschrieben, werden 700 mg Dexamethason-17-methylcarbonat mit

1) 0,2 ml Acetylchlorid,
2) 0,3 ml Buttersäurechlorid,
3) 0,4 ml Valeransäurechlorid und
4) 1 ml Cyclopentylpropionsäurechlorid anstatt des Propionsäurechlorids umgesetzt und aufgearbeitet.

Man erhält jeweils das entsprechende

1) Dexamethason-17-methylcarbonat-21-acetat,
2) -21-butyrat,
3) -21-valerat,
4) -21-cyclopentylpropionat

Beispiel 4

a) Zu einer Lösung von 1 g Dexamethason-17-n-propylcarbonat in 8 ml Dioxan und 4 ml Pyridin tropft man bei 0°C und unter Rühren eine Lösung von 0,8 ml Chlorameisensäuremethylester in 1 ml Dioxan und rührt das Reaktionsgemisch 16 Stunden bei 22°C. Danach gießt man in 250 ml halbgesättigte wässrige Kochsalzlösung ein, filtriert vom ausgefallenem Niederschlag ab, wäscht und trocknet ihn DC: $R_f = 0{,}60$. Bei einem weiteren analogen Ansatz wird der Niederschlag mit Methylenchlorid aufgenommen bzw. extrahiert. Man wäscht mit Wasser und destilliert im Vakuum vom Lösungsmittel ab, wobei ein Rückstand hinterbleibt. DC: $R_f = 0{,}60$. Ausbeute in beiden Fällen jeweils 900 mg. Zur Reindarstellung wird das rohe Reaktionsprodukt an Kieselgel (Säule 3 x 7 cm) mit Methylenchlorid chromatographiert. Die Fraktionen, in denen aufgrund des DC-Diagramms ausschließlich das gewünschte Reaktionsprodukt nachgewiesen wird (DC: $R_f = 0{,}60$), werden vereinigt und aus Aceton/Äther kristallisiert. Man erhält 720 mg Dexamethason-17-n-propylcarbonat-21-methylcarbonat vom Schmp. 167°C.

*IR*: 3440, 1730, 1655, 1610, 1440, 1265 $cm^{-1}$

MS-Spektrum; $M^+ = 536$

b) In gleicher Weise, wie in Beispiel 4 a) beschrieben, werden 1 g Dexamethason-17-n-propylcarbonat mit

1) 0,9 ml Chlorameisensäure-äthylester,
2) 1,0 ml Chlorameisensäure-n-propylester,
3) 1,1 ml Chlorameisensäure-n-butylester,
4) 1,0 ml Chlorameisensäure-iso-propylester,
5) 1,1 ml Chlorameisensäure-iso-butylester,
6) 0,8 ml Acetylchlorid,
7) 0,8 ml Propionsäurechlorid,
8) 0,9 ml Buttersäurechlorid,

9) 1 ml Valeriansäurechlorid,
10) 1 ml Cyclopropancarbonsäurechlorid und
11) 1,3 ml Cyclopentylpropionsäurechlorid statt des Chlorameisensäuremethylesters umgesetzt und aufgearbeitet.

Man erhält jeweils das entsprechende
1) *Dexamethason-17-n-propylcarbonat*-21-äthylcarbonat,
2) -21-n-propylcarbonat,
3) -21-n-butylcarbonat,
4) -21-iso-propylcarbonat,
5) 21-iso-butylcarbonat,
6) -21-acetat,
7) -21-propionat,
8) -21-butyrat,
9) -21-valerat,
10) -21-cyclopropancarbonsäureester,
11) -21-cyclopentylpropionat

c) In gleicher Weise, wie in Beispiel 2 f) beschrieben, werden 3 g Dexamethason-17-n-propylcarbonat mit Methansulfonsäurechlorid umgesetzt und aufgearbeitet. Man erhält nach dem Kristallisieren aus Aceton/Äther das Dexamethason-17-n-propylcarbonat-21-methansulfonat

Beispiel 5

a) In gleicher Weise, wie in Beispiel 4 a) beschrieben, werden 1 g Dexamethason-17-n-butylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende.
1) *Dexamethason-17-n-butylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g Dexamethason-17-n-butylcarbonat mit Methansulfonsäurechlorid umgesetzt und aufgearbeitet. Man erhält nach dem Kristallisieren aus Äther das Dexamethason-17-n-butylcarbonat-21-methansulfonat

Beispiel 6

a) In gleicher Weise, wie in Beispiel 4 a) beschrieben, werden 1 g Dexamethason-17-valerylcarbonat mit
1) 0,8 ml Chlorameisensäuremethylester,
2) 0,9 ml Chlorameisensäureäthylester,
3) 1,0 ml Chlorameisensäure-n-propylester,
4) 1,0 ml Chlorameisensäure-n-butylester,
5) 1,0 ml Chlorameisensäure-valerylester,
6) 0,8 ml Acetylchlorid,
7) 0,8 ml Propionsäurechlorid,

8) 0,9 ml Valeriansäurechlorid,

9) 1 ml Cyclopropancarbonsäurechlorid statt des Chlorameisensäuremethylesters umgesetzt und aufgearbeitet.

Man erhält jeweils das entsprechende

1) *Dexamethason-17-valerylcarbonat*-21-methylcarbonat,

2) -21-äthylcarbonat,

3) -21-n-propylcarbonat,

4) -21-n-butylcarbonat,

5) -21-valerylcarbonat,

6) -21-acetat,

7) -21-propionat,

8) -21-valerat,

9) -21-cyclopropancarbonsäureester

b) In gleicher Weise, wie in Beispiel 2 f) beschrieben, werden 3 g Dexamethason-17-valerylcarbonat mit Methansulfonsäurechlorid umgesetzt und aufgearbeitet. Man erhält so das Dexamethason-17-valerylcarbonat-21-methansulfonat.

Beispiel 7

6α-Fluor-prednisolon-17α-äthylcarbonat-21-propionat

5,4 g 6α-Fluor-prednisolon-17α,21-diäthyl-orthocarbonat werden in 200 ml Eisessig und 2 ml Wasser 5 Stunden lang bei 20° gerührt. Anschliessend wird das Reaktionsgemisch in 1.5 l halbgesättigte Kochsalzlösung eingerührt. Der hierbei ausfallende Niederschlag wird abfiltriert, mit Wasser gewaschen und getrocknet.

Die so erhaltenen 4,45 g 6α-Fluor-prednisolon-17α-äthylcarbonat vom Schmp. 133—136° können ohne weitere Reinigung wie folgt sogleich weiter umgesetzt werden. Hierzu wird obige Substanz in 60 ml absoluten Pyridin gelöst und nach Abkühlen auf 0° mit 2.3 ml Propionsäurechlorid versetzt. Nach 1 Stunde bei 0° und einer weiteren Stunde bei 20° wird das Reaktionsgemisch in 500 ml halbgesättigte wässrige Kochsalzlösung eingerührt. Anschliessend wird mit Methylenchlorid extrahiert, die organische Phase mit Wasser, verdünnten Salzsäure und Wasser neutral gewaschen, getrocknet und im Vakuum zur Trocknen eingedampft. Die erhaltenen 4,95 g rohes 6α - Fluor - prednisolon - 17α - äthylcarbonat - 21 - propionat können wie folgt gereinigt werden.

Hierzu wird wie in Beispiel 2 a beschrieben an einer Säule aus 250 g Kieselgel chromatographiert und aufgearbeitet. Am Schluß wird hier aus Äther/Petroläther umkristallisiert und man erhält 2,55 g 6α - Fluor - prednisolon - 17α - äthylcarbonat - 21 - propionat vom Schmp. 147—148°.

Das als Ausgangsmaterial verwendete 6α-Fluor-prednisolon-17α,21-diäthyl-orthocarbonat wird analog gemäss DBP 16 68 079 wie folgt erhalten.

Eine Lösung von 4,75 g 6α-Fluor-prednisolon in 180 ml Wasserfreien Dioxan wird nach Zugabe von 13 ml Tetraäthylorthocarbonat und 0 29 g p-Toluolsulfonsäure 15 Stunden lang bei Raumtemperatur gerührt. Dann wird das Reaktionsgemisch in eine Lösung von 1,5 g Natriumhydrogencarbonat in 950 ml Wasser eingegossen. Die ausgefallenen Kristalle werden gesammelt, mit Wasser gewaschen, getrocknet und aus Aceton umkristallisiert.

Es werden 4,1 g 6α - Fluor - prednisolon - 17α,21 - diäthylorthocarbonat vom Schmp, 178—180° erhalten.

Beispiel 8

6α-Fluor-prednisolon-17α-äthylcarbonat-21-chloracetat

4 g 6α-Fluor-prednisolon-17α-äthylcarbonat werden in 110 ml abs. Tetrahydrofuran gelöst, mit 1,69 g Chloressigsäureanhydrid und 0,65 ml abs. Pyridin versetzt und 28 Stunden bei Raumtemperatur gerührt.

Danach wurden 20 ml Wasser zugegeben, im Vakuum und H.V. bei 40° Badtemperatur zur Trockne einrotiert. Der Rückstand wird in 150 ml Essigester aufgenommen, mit 12 ml 2n Salzsäure, Wasser, verdünnter Natriumhydrogencarbonatlösung und Wasser neutral gewaschen und die organische Phase über Natriumsulfat getrocknet und im Vakuum abermals zur Trockne eingedampft. Der Rückstand wird aus Diisopropyläther/Petroläther umkristallisiert.

Es werden 3,9 g 6α-Fluor-prednisolon-17α-äthylcarbonat-21-chloracetat vom Schmp. 134—138° erhalten.

Beispiel 9

6α-Fluor-prednisolon-17α-äthylcarbonat-21-morpholinacetat-hydrochlorid

0,5 g 6α-Fluor-prednisolon-17α-äthylcarbonat-21-chloracetat werden mit 0,4 ml Morpholin in 16 ml Aceton 3 Stunden lang unter Rückfluss zum Sieden erhitzt. Dann wird im Vakuum eingedampft, der Rückstand in 10 ml Essigester gelöst und 3 mal mit je 15,5 ml 0,10 n Salzsäure ausgeschüttelt. Anschliessend werden die vereinigten wässrigen Phasen mit Natriumhydrogencarbonatlösung schwach alkalisch gestellt. Der ausgefallene Niederschlag wird gesammelt, mit etwas Wasser gewaschen, in Essigester gelöst, über Natriumsulfat getrocknet und im V. zur Trockne eingedampft. Der Rückstand wird in 5 ml abs. Äthanol aufgenommen, mit 3,2 ml 0,30 n Chlorwasserstoff in abs. Äthanol versetzt.

Anschliessend wird abermals im Vakuum zur Trockne eingeengt und mit Hexan zur Kristallisation gebracht.

Es werden 290 mg 6$\alpha$-Fluor-prednisolon-17$\alpha$-äthylcarbonat-21-morpholinacetat-hydrochlorid von Schmp. 185—188° erhalten.

Beispiel 10

6$\alpha$-Methyl-prednisolon-17$\alpha$päthylcarbonat

21,0 g 6$\alpha$-Methyl-prednisolon-17$\alpha$,21-diäthyl-orthocarbonat werden in einer Mischung aus 700 ml Eisessig und 1,0 ml Wasser 2 Stunden lang bei Raumtemperatur gerührt. Dann wird in 3,0 l Eiswasser gegossen, mit 875 ml konzentrierter wässrigen Ammoniaklösung neutralisiert, der ausgefallene Niederschlag abgesaugt und mit etwas Wasser gewaschen. Die vereinigten Filtrate wurden mit Methylenchlorid extrahiert und der obige Filterrückstand in der organischen Phase gelöst, die selbe über Natriumsulfat getrocknet und im Vakuum zur Trockne eingedampft. Nach dem Umkristallisieren aus wenig Methylenchlorid und Äther werden 16,7 g 6$\alpha$-Methyl-prednisolon-17$\alpha$-äthylcarbonat vom Schmp. 188—190° erhalten. Das als Ausgangsmaterial verwendete 6$\alpha$-Methyl-prednisolon-17$\alpha$,21-diäthylcarbonat wird analog gemäss DBP 16 68 079 wie folgt erhalten. Eine Lösung von 17 g Urbason in 600 ml wasserfreien Dioxan wird nach Zugabe von 47,0 ml Tetraäthylorthocarbonat und 1,05 g p-Toluolsulfonsäure 5 Stunden lang bei Raumtemperatur gerührt. Dann wird das Reaktionsgemisch in eine Lösung von 6,0 g Natriumhydrogencarbonat in 4,0 l Wasser eingegossen. Dann wird wie unter Beispiel 7 beschrieben aufgearbeitet. Es wurden 21,1 g 6$\alpha$ - Methyl - prednisolon - 17$\alpha$,21 - bis - (äthylcarbonat) vom Schmp. 109—112° erhalten.

Beispiel 11

6$\alpha$-Methyl-prednisolon-17$\alpha$-äthylcarbonat-21-methylcarbonat

8,5 g 6$\alpha$-Methyl-prednisolon-17$\alpha$-äthylcarbonat werden in einer Mischung aus 85 ml wasserfreien Dioxan und 42 ml wasserfreien Pyridin gelöst. Dann werden bei 0° unter Eiskühlung und Rühren 7,2 ml Chlorameisensäuremethylester zugetropft. Nach 15 Stunden langem Stehen bei 0° wird in 800 ml halbkonzentrierten wässrigen Kochsalzlösung eingerührt. Nach 3 stündigen Stehen werden die ausgefallenen Kristalle gesammelt, mit Wasser gewaschen und im Vakuum bei 60° getrocknet.

Nach dem Umkristallisieren aus Diisopropyläther/Hexan werden 8,2 g 6$\alpha$ - Methyl - prednisolon - 17$\alpha$ - äthylcarbonat - 21 - methylcarbonat vom Schmp. 121—123° erhalten.

Beispiel 12

6$\alpha$-Methyl-prednisolon-17$\alpha$,21-bis-(äthylcarbonat)

Zu einer eiskalten Lösung von 3,2 g 6$\alpha$-Methyl-prednisolon-17$\alpha$-äthylcarbonat in 10,5 ml wasserfreien Pyridin wurde unter Eiskühlung eine Lösung von 2,3 g (=2 ml) Chlorameisensäureäthylester in 18 meabs. Dioxan tropfenweise unter Rühren gegen. Nach 4 1/2 Stunden bei 0° wurde das Reaktionsgemisch in 200 ml halbgesättigte Kochsalzlosung eingerührt. Dann wurde 3 mal mit je 100 ml Methylenchlorid ausgeschüttelt. Die vereinten organischen Phasen wurden mit 0,5 n Salzsäure und Wasser neutral gewaschen, über Natriumsulfat getrocknet und im Vakuum z.Tr. eingeengt. Es werden 3,15 g rohes 6$\alpha$ - Methyl - prednisolon - 17$\alpha$,21 - bis - (äthylcarbonat) vom Schmp. 136—140° erhalten. Zur weiteren Reinigung wird an einer 4 x 10 cm grossen Säule aus Kieselgel mit Methylenchlorid als Laufmittel fraktioniert chromatographiert. Die im Dünnschicht einheitlichen Fraktionen wurden vereinigt und im Vakuum zur Trockne eingedampft. Nach dem Digerieren mit Diisopropyläther werden 2,3 g 6$\alpha$ - Methyl - prednisolon - 17$\alpha$,21 - bis - (äthylcarbonat) vom Schmp. 142—143° erhalten.

Beispiel 13

6$\alpha$-Methyl-prednisolon-17$\alpha$-äthylcarbonat-21-propionat

Zu einer Lösung von 3,0 g 6$\alpha$-Methyl-prednisolon-17$\alpha$-äthylcarbonat in 36 ml Pyridin werden unter Eiskühlung 0,78 ml Propionsäurechlorid eingerührt.

Es wurde anschliessend 30 Minuten bei 0°, dann 1 Stunde bei Raumtemperatur gerührt und das Reaktionsgemisch schliesslich in 200 ml wässrige Kochsalzlösung eingegossen. Weiter wurde wie in Beispiel 12 beschrieben aufgearbeitet.

Nach der Chromatographie und der Kristallisation mit Diisopropyläther wurden 2,4 g 6$\alpha$ - Methyl - prednisolon - 17$\alpha$ - äthyl - carbonat - 21 - propionat vom Schmp. 156—158° erhalten.

Beispiel 14

6$\alpha$-Methyl-prednisolon-17$\alpha$-äthylcarbonat-21-cyclopropancarbonsäureester

Zu einer Lösung von 5,23 g 6$\alpha$-Methyl-prednisolon-17$\alpha$-äthylcarbonat in 60 ml abs. Pyridin werden unter Eiskühlung 1,0 ml Cyclopropancarbonsäurechlorid eingerührt. Es wurde 30 Min bei 0° gerührt und dann noch 16 Stunden bei Raumtemperatur sich selbst überlassen. Nach Einrühren in 350 ml Wasser wird in Beispiel 12 beschrieben, aufgearbeitet und chromatographiert (an 180 g Kieselgel). Nach dem Umkristallisieren aus Diisopropyläther (Hexan werden 3,93 g 6$\alpha$ - Methyl - prednisolon - 17$\alpha$ - äthylcarbonat - 21 - cyclopropancarbonsäureester vom Schmp. 167—170° erhalten.

Beispiel 15

6α-Methyl-prednisolon-17α-äthylcarbonat-21-(1-adamantoat)

Zu einer Lösung von 2,0 g 6α-Methyl-prednisolon in 130 ml Toluol werden 1,73 g Adamantancarbonsäurechlorid und 1,0 ml abs. Pyridin gegeben und dann 15 Stunden lang unter Rückfluss zum Sieden erhitzt. Es wird auf Raumtemperatur abgekühlt und mit Natriumhydrogencarbonatlösung und dann neutral gewaschen, über Natriumsulfat getrocknet und im Vakuum zur Trockne eingeengt.

Der Rückstand wird an 100 g Kieselgel mittels Toluol/Essigester 3:1 chromatographiert (vergl, Beispiel 12). Nach dem Digerieren mit Äther werden 1,1 g 6α - Methyl - prednisolon - 17α - äthylcarbonat - 21 - (1 - adamantoat)vom Schmp. 265—266° erhalten.

Beispiel 16

6α-Methyl-prednisolon-17α-äthylcarbonat-21-cyclopentylpropionat

Zu einer 30°C warmen Lösung von 5,0 g 6α-Methyl-prednisolon-17α-äthylcarbonat in einer Mischung aus 23 ml wassertreien Pyridin und 25 ml wasserfreien Aceton werden unter Stickstoff innerhalb von 30 Minuten 6,4 g Cyclopentylpropionsäurechlorid unter Rühren zugetropft. Danach wird noch eine Stunde bei 46—48° nachgerührt. Dann wurden bei dieser Temperatur innerhalb von 5 Minuten 2,17 ml Diäthylaminoäthanol unter Rühren zugetropft und 20 Minuten nach gerührt. Dann wird auf 20° abgekühlt und innerhalb 20 Minuten 30 ml Wasser eingerührt. Nach weiteren 20 Minuten langem Rühren wird das organische Lösungsmittel im Vakuum abgedampft und anschliessend wie in Beispiel 12 beschrieben, aufgearbeitet und chromatographiert.

Es werden 2,6 g 6α-Methyl-prednisolon-17α-äthylcarbonat-21-cyclopentylpropionat vom Schmp. 175—176° erhalten.

Beispiel 17

a) In gleicher Weise, wie in Beispiel 4 a) beschrieben, werden 1 g Prednisolon-17-methylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt udn aufgearbeitet.

Man erhält jeweils das entsprechende

1) *Prednisolon-17-methylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g Prednisolon-17-methylcarbonat mit Methansulfonsäurechlorid umgesetzt und aufgearbeitet. Man erhält nach dem Kristallisieren aus Äther das Prednisolon-17-methylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende Prednisolon-17-methylcarbonat-21-p-toluolsulfonat bzw. -21-p-chlorbenzolsulfonat.

c) Das für die Reaktion zunächst benötigte Prednisolon-dimethylorthocarbonat ($R_F$≅0,6) wird gemäß DBP 1 668 079 aus Prednisolon und Tetra-methyl-orthocarbonat hergestellt.

Anschließend wird ersteres in gleicher Weise, wie in Beispiel 1 c) beschrieben, zu Prednisolon-17-methyl carbonat ($R_F$≅0,4) hydrolisiert.

Beispiel 18

a) In gleicher Weise, wie in Beispiel 4a) beschrieben, werden 1 g Prednisolon-17-methylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *Prednison-17-methylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g Prednison-17-methylcarbonat mit Methansulfonsäurechlorid umgesetzt und aufgearbeitet. Man erhält nach Kristallisieren aus Ather das Prednison-17-methylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende Prednison-17-methylcarbonat-21-p-toluolsulfonat bzw. -21-p-chlorbenzolsulfonat.

c) Das für die Reaktion zunächst benötigte Prednison-dimethyl-orthocarbonat ($R_F \cong 0,6$) wird gemäß DBP 1 668 079 aus Prednison und Tetramethylorthocarbonat hergestellt.

Anschließend wird ersteres in gleicher Weise, wie in Beispiel 1 c) beschrieben, zu Prednison-17-methylcarbonat ($R_F \cong 0,4$) hydrolisiert.

Beispiel 19

a) In gleicher Weise, wie in Beispiel 4 a) beschrieben, werden 1 g Cortison-17-methylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *Cortison-17-methylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,

10) -21-valerat,
11) -21-cyclopropancarbonsäureester,
12) -21-cyclopentylpropionat

b) In gleicher Weise, wie in Beispiel 2 f) beschrieben, werden 3 g Cortison-17-methylcarbonat mit Methansulfonsäurechlorid umgesetzt und aufgearbeitet. Man erhält nach dem Kristallisieren aus Äther das Cortison-17-methylcarbonat-21-methansulfonat.

Wird anstatt des Methanesulfonsäurechlorids eine äquimolare Menge des p-Toluolsulfonsäure- oder des p-Chlorbenzolsulfonsäurechlorids eingesetzt, so erhält man das entsprechende Cortison-17-methylcarbonat-21-p-toluolsulfonat bzw. -21-p-chlorbenzolsulfonat.

c) Das für die Reaktion zunächst benötigte Cortison-dimethyl-orthocarbonat ($R_F \cong 0{,}6$) wird gemäß DBP 1 668 079 aus Cortison und Tetramethylorthocarbonat hergestellt.

Anschließend wird ersteres in gleicher Weise, wie in Beispiel 1 c) beschrieben, zu Cortison-17-methylcarbonat ($R_F \cong 0{,}4$) hydrolisiert.

Beispiel 20

a) In gleicher Weise, wie in Beispiel 4 a) beschrieben, werden 1 g Cortisol-17-methylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende
1) *Cortisol-17-methylcarbonat-*21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g Cortisol-17-methylcarbonat mit Methansulfonsäurechlorid umgesetzt und aufgearbeitet. Man erhält nach dem Kristallisieren aus Äther das Cortisol-17-methylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende Cortisol-17-methylcarbonat-21-p-toluolsulfonat bzw. -21-p-chlorbenzolsulfonat.

c) Das für die Reaktion zunächst benötigte Cortisol-dimethyl-orthocarbonat ($R_F \cong 0{,}6$) wird gemäß DBP 1 668 079 aus Cortisol und Tetramethylorthocarbonat hergestellt.

Anschließend wird ersteres in gleicher Weise, wie in Beispiel 1 c) beschrieben, zu Cortisol-17-methylcarbonat ($R_F \cong 0{,}4$) hydrolisiert.

Beispiel 21

a) In gleicher Weise, wie in Beispiel 4 a) beschrieben, werden 1 g Beclomethason-17-methylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,

10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende
1) *Beclomethason-17-methylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g Beclomethason-17-methylcarbonat mit Methansulfonsäurechlorid umgesetzt und aufgearbeitet. Man erhält nach dem Kristallisieren aus Äther das Beclomethason-17-methylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende Beclomethason-17-methylcarbonat-21-p-toluolsulfonat bzw. -21-p-chlorbenzolsulfonat.

c) Das für die Reaktion zunächst benötigte Beclomethason-dimethyl-orthocarbonat ($R_F \cong 0,6$) wird gemäß DBP 1 668 079 aus Beclomethason und Tetramethylorthocarbonat hergestellt.

Anschließend wird ersteres in gleicher Weise, wie in Beispiel 1 c) beschrieben, zu Beclomethason-17-methylcarbonat ($R_F \cong 0,4$) hydrolisiert.

Beispiel 22

a) In gleicher Weise, wie in Beispiel 4 a) beschrieben, werden 1 g 6α-Fluordexamethason-17-methylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende
1) *6α-Fluordexamethason-17-methylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g 6α-Fluordexamethason-17-methylcarbonat mit Methansulfonsäurechlorid umgesetzt und aufgearbeitet. Man erhält nach dem Kristallisieren aus Äther das 6α-Fluordexamethason-17-methylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende 6α-Fluordexamethason-17-methylcarbonat-21-p-toluolsulfonat bzw. -21-p-chlorbenzolsulfonat.

c) Das für die Reaktion zunächst benötigte 6α-Fluordexamethason-dimethyl-orthocarbonat

($R_F \cong 0{,}6$) wird gemäß DBP 1 668 079 aus 6α-Fluordexamethason und Tetramethylorthocarbonat hergestellt.

Anschließend wird ersteres in gleicher Weise, wie in Beispiel 1 c) beschrieben, zu 6α-Fluordexamethason-17-methylcarbonat ($R_F \cong 0{,}4$) hydrolisiert.

Beispiel 23

a) In gleicher Weise, wie in Beispiel 4 a) beschrieben, werden 1 g Betamethason-17-methylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *Betamethason-17-methylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g Betamethason-17-methylcarbonat mit Methansulfonsäurechlorid umgesetzt und aufgearbeitet. Man erhält nach dem Kristallisieren aus Äther das Betamethason-17-methylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende Betamethason-17-methylcarbonat-21-p-toluolsulfonat bzw. -21-p-chlorbenzolsulfonat.

c) Das für die Reaktion zunächst benötigte Betamethason-dimethyl-orthocarbonat ($R_F \cong 0{,}6$) wird gemäß DBP 1 668 079 aus Betamethason und Tetra methylorthocarbonat hergestellt.

Anschließend wird ersteres in gleicher Weise, wie in Beispiel 1 c) beschrieben, zu Betamethason-17-methylcarbonat ($R_F \cong 0{,}4$) hydrolisiert.

Beispiel 24

a) In gleicher Weise, wie in Beispiel 4 a) beschrieben, werden 1 g 6α-Fluor-Prednisolon-17-methylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende
1) *6α-Fluor-Prednisolon-17-methyl-carbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g 6α-Fluor-Prednisolon-17-methylcarbonat mit Methansulfonsäurechlorid umgesetzt und aufgearbeitet. Man erhält nach dem Kristallisieren aus Äther das 6α-Fluor-prednisolon-17-methylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende 6α-Fluor-prednisolon-17-methylcarbonat-21-p-toluolsulfonat bzw. -21-p-chlorbenzolsulfonat.

c) Das für die Reaktion zunächst benötigte 6α-Fluor-Prednisolon-dimethyl-orthocarbonat ($R_F \cong 0{,}6$) wird gemäß DBP 1 668 079 aus 6α-Fluor-Prednisolon und Tetra-methylorthocarbonat hergestellt.

Anschließend wird ersteres in gleicher Weise, wie in Beispiel 1 c) beschrieben, zu 6α-Fluor-prednisolon-17-methylcarbonat ($R_F \cong 0{,}4$) hydrolisiert.

Beispiel 25

a) In gleicher Weise, wie in Beispiel 4 a) beschrieben, werden 1 g 16α- oder β-Methyl-prednisolon-17-methylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende
1) *16α- oder β-Methylprednisolon-17-methylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g 16α- oder β-Methyl-prednisolon-17-methylcarbonat mit Methansulfonsäurechlorid umgesetzt und aufgearbeitet. Man erhält nach dem Kristallisieren aus Äther das 16α- oder β-Methylcarbonat-17-methylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende 16α- oder β-Methylprednisolon-17-methylcarbonat-21-p-toluolsulfonat bzw. -21-p-chlorbenzolsulfonat.

c) Das für die Reaktion zunächst benötigte 16α- der β-Methylprednisolon-dimethyl-orthocarbonat ($R_F \cong 0{,}6$) wird gemäß DBP 1 668 079 aus 16α- oder β-Methylprednisolon und Tetramethylorthocarbonat hergestellt.

Anschließend wird ersteres in gleicher Weise, wie in Beispiel 1 c) beschrieben, zu 16α- oder β-Methylprednisolon-17-methylcarbonat ($R_F \cong 0,4$) hydrolisiert.

Beispiel 26

a) In gleicher Weise, wie in Beispiel 4 a) beschrieben, werden 1 g 6α, 16α- oder β-Dimethyl-prednisolon-17-methylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *6, 16α- oder β-Dimethyl-prednisolon-17*-methylcarbonat-21-methylcarbonat,
2) -21-n-propylcarbonat,
3) -21-n-propylcarbonat,
4) -21-n-butylcarbonat,
5) -21-iso-butylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g 6α, 16α- oder β-Dimethyl-prednisolon-17-methylcarbonat mit Methansulfonsäurechlorid umgesetzt und aufgearbeitet. Man erhält nach dem Kristallisieren aus Äther das 6α, 16α- oder β-Dimethyl-prednisolon-17-methylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende 6α, 16α- oder β-Dimethyl-prednisolon-17-methylcarbonat-21-p-toluolsulfonat bzw. -21-p-chlorbenzolsulfonat.

c) Das für die Reaktion zunächst benötigte 6α, 16α- oder β-Dimethyl-prednisolon-dimethyl-orthocarbonat ($R_F \cong 0,6$) wird gemäß DBP 1 668 079 aus 6α, 16α- oder β-Dimethyl-prednisolon und Tetramethylorthocarbonat hergestellt.

Anschließend wird ersteres in gleicher Weise, wie in Beispiel 1 c) beschrieben, zu 6α, 16α- oder β-Dimethyl-prednisolon-17-methylcarbonat ($R_F \cong 0,4$) hydrolisiert.

Beispiel 27

a) In gleicher Weise, wie in Beispiel 4 a) beschrieben, werden 1 g 9α-Chlor, 16α-methyl-prednisolon-17-methylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *9α-Chlor, 16α-methyl-prednisolon-17-methylcarbonat*-21-methylcarbonat,
2) -21-äthylcarbonat,
3) -21-n-propylcarbonat,

4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat,

b) In gleicher Weise, wie in Beispiel 2 f) beschrieben, werden 3 g 9α-Chlor, 16α-methyl-prednisolon-17-methylcarbonat mit Methansulfonsäurechlorid umgesetzt und aufgearbeitet. Man erhält nach dem Kristallisieren aus Äther das 9α-Chlor, 16α-Methyl-prednisolon-17-methylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende 9α-Chlor, 16α-methyl-prednisolon-17-methylcarbonat-21-p-toluolsulfonat bzw. -21-p-chlorbenzolsulfonat.

c) Das für die Reaktion zunächst benötigte 9α-Chlor, 16α-methyl-prednisolon-dimethyl-orthocarbonat ($R_F \cong 0,6$) wird gemäß DBP 1 668 079 aus 9α-Chlor, 16α-methyl-prednisolon und Tetramethyl-orthocarbonat hergestellt.

Anschließend wird ersteres in gleicher Weise, wie in Beispiel 1 c) beschrieben, zu 9α-Chlor, 16α-methyl-prednisolon-17-methylcarbonat ($R_F \cong 0,4$) hydrolisiert.

Beispiel 28

a) In gleicher Weise, wie in Beispiel 4 a) beschrieben, werden 1 g 9α-Chlor-prednisolon-17-methylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende
1) *9α-Chlor-prednisolon-17-methylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g 9α-Chlor-prednisolon-17-methylcarbonat mit Methansulfonsäurechlorid umgesetzt und aufgearbeitet. Man erhält nach dem Kristallisieren aus Äther das 9α-Chlor-prednisolon-17-methylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende 9α-Chlor-prednisolon-17-methylcarbonat-21-p-toluolsulfonat bzw. -21-p-chlorbenzolsulfonat.

c) Das für die Reaktion zunächst benötigte 9α-Chlor-prednisolon-dimethyl-orthocarbonat ($R_F \cong 0,6$) wird gemäß DBP 1 668 079 aus 9α-Chlor-prednisolon und Tetra-methyl-orthocarbonat hergestellt.

Anschließend wird ersteres in gleicher Weise, wie in Beispiel 1 c) beschrieben, zu 9α-Chlor-prednisolon-17-methylcarbonat ($R_F \cong 0,4$) hydrolisiert.

Beispiel 29

a) In gleicher Weise, wie in Beispiel 4 a) beschrieben, werden 1 g Prednisolon-17-äthylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *Prednisolon-17-äthylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g Prednisolon-17-äthylcarbonat mit Methansulfonsäurechlorid umgesetzt und aufgearbeitet. Man erhält nach dem Kristallisieren aus Äther das Prednisolon-17-äthylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende Prednisolon-17-äthylcarbonat-21-p-toluolsulfonat bzw. -21-p-chlorbenzolsulfonat.

c) Das für die Reaktion zunächst benötigte Prednisolon-diäthyl-orthocarbonat ($R_F \cong 0,6$) wird gemäß DBP 1 668 079 aus Prednisolon und Tetra-äthylorthocarbonat hergestellt.

Anschließend wird ersteres in gleicher Weise, wie in Beispiel 1 c) beschrieben, zu Prednisolon-17-äthyl-carbonat ($R_F \cong 0,4$) hydrolisiert.

Beispiel 30

a) In gleicher Weise, wie in Beispiel 4 a) beschrieben, werden 1 g Prednison-17-äthylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *Prednison-17-äthylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,

9) -21-butyrat,
10) -21-valerat,
11) -21-cyclopropancarbonsäureester,
12) -21-cyclopentylpropionat,

b) In gleicher Weise, wie in Beispiel 2 f) beschrieben, werden 3 g Prednison-17-äthylcarbonat mit Methansulfonsäurechlorid umgesetzt und aufgearbeitet. Man erhält nach dem Kristallisieren aus Äther das Prednison-17-äthylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende Prednison-17-äthylcarbonat-21-p-toluolsulfonat bzw. -21-p-chlorbenzolsulfonat.

c) Das für die Reaktion zunächst benötigte-Prednison-diäthyl-orthocarbonat ($R_F \cong 0,6$) wird gemäß DBP 1 668 079 aus Prednison und Tetra-äthylorthocarbonat hergestellt.

Anschließend wird ersteres in gleicher Weise, wie in Beispiel 1 c) beschrieben, zu Prednison-17-äthylcarbonat ($R_F \cong 0,4$) hydrolisiert.

Beispiel 31

a) In gleicher Weise, wie in Beispiel 4 a) beschrieben, werden 1 g Cortison-17-äthylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende
1) *Cortison-17-äthylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g Cortison-17-äthylcarbonat mit Methansulfonsäurechlorid umgesetzt und aufgearbeitet. Man erhält nach dem Kristallisieren aus Äther das Cortison-17-äthylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende Cortison-17-äthylcarbonat-21-p-toluolsulfonat bzw. -21-p-chlorbenzolsulfonat.

c) Das für die Reaktion zunächst benötigte Cortison-diäthyl-orthocarbonat ($R_F \cong 0,6$) wird gemäß DBP 1 668 079 aus Cortison und Tetra-äthylorthocarbonat hergestellt.

Anschließend wird ersteres in gleicher Weise, wie in Beispiel 1 c) beschrieben, zu Cortison-17-äthylcarbonat ($R_F \cong 0,4$) hydrolisiert.

Beispiel 32

a) In gleicher Weise, wie in Beispiel 4 a) beschrieben, werden 1g Cortisol-17-äthylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,

10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende
1) *Cortisol-17-äthylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g Cortisol-17-äthylcarbonat mit Methansulfonsäurechlorid umgesetzt und aufgearbeitet. Man erhält nach dem Kristallisieren aus Äther das Cortisol-17-äthylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende Cortisol-17-äthylcarbonat-21-p-toluolsulfonat bzw. -21-p-chlorbenzolsulfonat.

c) Das für die Reaktion zunächst benötigte Cortisol-diäthyl-orthocarbonat ($R_F \cong 0,6$) wird gemäß DBP 1 668 079 aus Cortisol und Tetra-äthyl-orthocarbonat hergestellt.

Anschließend wird ersteres in gleicher Weise, wie in Beispiel 1 c) beschrieben, zu Cortisol-17-äthylcarbonat ($R_F \cong 0,4$) hydrolisiert.

## Beispiel 33

a) In gleicher Weise, wie in Beispiel 4 a) beschrieben, werden 1 g Beclomethason-17-äthylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende
1) *Beclomethason-17-äthylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g Beclomethason-17-äthylcarbonat mit Methansulfonsäurechlorid umgesetzt und aufgearbeitet. Man erhält nach dem Kristallisieren aus Äther das Beclomethason-17-äthylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende Beclomethason-17-äthylcarbonat-21-p-toluolsulfonat bzw. -21-p-chlorbenzolsulfonat.

c) Das für die Reaktion zunächst benötigte Beclomethason-diäthyl-orthocarbonat ($R_F \cong 0{,}6$) wird gemäß DBP 1 668 079 aus Beclomethason und Tetra-äthyl-orthocarbonat hergestellt.

Anschließend wird ersteres in gleicher Weise, wie in Beispiel 1 c) beschrieben, zu Beclomethason-17-äthylcarbonat ($R_F \cong 0{,}4$) hydrolisiert.

Beispiel 34

a) In gleicher Weise, wie in Beispiel 4 a) beschrieben, werden 1 g 6$\alpha$-Fluordexamethason-17-äthylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *6α-Fluordexamethason-17-äthylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g 6$\alpha$-Fluordexamethason-17-äthylcarbonat mit Methansulfonsäurechlorid umgesetzt und aufgearbeitet. Man erhält nach dem Kristallisieren aus Äther das 6$\alpha$-Fluordexamethason-17-äthylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende 6$\alpha$-Fluordexamethason-17-äthylcarbonat-21-p-toluolsulfonat bzw. -21-p-chlorbenzolsulfonat.

c) Das für die Reaktion zunächst benötigte 6$\alpha$-Fluordexamethason-di-äthyl-orthocarbonat ($R_F \cong 0{,}6$) wird gemäß DBP 1 668 079 aus 6$\alpha$-Fluordexamethason und Tetra-äthyl-orthocarbonat hergestellt.

Anschließend wird ersteres in gleicher Weise, wie in Beispiel 1 c) beschrieben, zu 6$\alpha$-Fluordexamethason-17-äthylcarbonat ($R_F \cong 0{,}4$) hydrolisiert.

Beispiel 35

a) In gleicher Weise, wie in Beispiel 4 a) beschrieben, werden 1 g Betamethason-17-äthylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *Betamethason-17-äthylcarbonat*-21-methylcarbonat,
2) -21-äthylcarbonat,

3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g Betamethason-17-äthylcarbonat mit Methansulfonsäurechlorid umgesetzt und aufgearbeitet. Man erhält nach dem Kristallisieren aus Äther das Betamethason-17-äthylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende Betamethason-17-äthylcarbonat-21-p-toluolsulfonat bzw. -21-p-chlorbenzolsulfonat.

c) Das für die Reaktion zunächst benötigte Betamethason-diäthyl-orthocarbonat ($R_F \cong 0,6$) wird gemäß DBP 1 668 079 aus Betamethason und Tetra-äthyl-orthocarbonat hergestellt.

Anschließend wird ersteres in gleicher Weise, wie in Beispiel 1 c) beschrieben, zu Betamethason-17-äthylcarbonat ($R_F \cong 0,4$) hydrolisiert.

## Beispiel 36

a) In gleicher Weise, wie in Beispiel 4 a) beschrieben, werden 1 g 6$\alpha$-Fluor-prednisolon-17-äthylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *6$\alpha$-Fluorprednisolon-17-äthylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g 6$\alpha$-Fluorprednisolon-17-äthylcarbonat mit Methansulfonsäurechlorid umgesetzt und aufgearbeitet. Man erhält nach dem Kristallisieren aus Äther das 6$\alpha$-Fluor-prednisolon-17-äthylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende 6$\alpha$-Fluorprednisolon-17-äthylcarbonat-21-p-toluolsulfonat bzw. -21-p-chlorbenzolsulfonat.

c) Das für die Reaktion zunächst benötigte 6$\alpha$-Fluorprednisolon-di-äthyl-orthocarbonat ($R_F \cong 0,6$) wird gemäß DBP 1 668 079 aus 6$\alpha$-Fluor-prednisolon und Tetra-äthyl-orthocarbonat hergestellt.

Anschließend wird ersteres in gleicher Weise, wie in Beispiel 1 c) beschrieben, zu 6$\alpha$-Fluor-prednisolon-17-äthylcarbonat ($R_F \cong 0,4$) hydrolisiert.

Beispiel 37

a) In gleicher Weise, wie in Beispiel 4 a) beschrieben, werden 1 g 16α- oder β-Methyl-prednisolon-17-äthylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *16α- oder β-Methylprednisolon-17-äthylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g 16α- oder β-Methylprednisolon-17-äthylcarbonat mit Methansulfonsäurechlorid umgesetzt und aufgearbeitet. Man erhält nach dem Kristallisieren aus Äther das 16α- oder β-Methyl-prednisolon-17-äthylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende 16α- oder β-Methyl-prednisolon-17-äthylcarbonat-21-p-toluolsulfonat bzw. -21-p-chlorbenzolsulfonat.

c) Das für die Reaktion zunächst benötigte 16α- oder β-Methyl-prednisolon-di-äthyl-orthocarbonat ($R_F \cong 0{,}6$) wird gemäß DBP 1 668 079 aus 16α- oder β-Methyl-prednisolon und Tetra-äthylorthocarbonat hergestellt.

Anschließend wird ersteres in gleicher Weise, wie in Beispiel 1 c) beschrieben, zu 16α- oder β-Methyl-prednisolon-17-äthylcarbonat ($R_F \cong 0{,}4$) hydrolisiert.

Beispiel 38

a) In gleicher Weise, wie in Beispiel 4 a) beschrieben, werden 1 g 6α, 16α- oder β-Dimethylprednisolon-17-äthylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *6α, 16α, oder β-Dimethyl-predinisolon-17-äthylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,

7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g 6α, 16α- oder β-Dimethyl-prednisolon-17-äthylcarbonat mit Methansulfonsäurechlorid umgesetzt und aufgearbeitet. Man erhält nach dem Kristallisieren aus Äther das 6α, 16α- oder β-Dimethyl-prednisolon-17-äthylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende 6α, 16α- oder β-Dimethyl-prednisolon-17-äthylcarbonat-21-p-toluolsulfonat bzw. -21-p-chlorbenzolsulfonat.

c) Das für die Reaktion zunächst benötigte 6α, 16α- oder β-Dimethyl-prednisolon-diäthyl-orthocarbonat ($R_F \cong 0,6$) wird gemäß DBP 1 668 079 aus 6α, 16α- oder β-Dimethyl-prednisolon und Tetra-äthyl-orthocarbonat hergestellt.

Anschließend wird ersteres in gleicher Weise, wie in Beispiel 1 c) beschrieben, zu 6α, 16α- oder β-Dimethyl-prednisolon-17-äthyl-carbonat ($R_f \cong 0,4$) hydrolisiert.

## Beispiel 39

a) In gleicher Weise, wie in Beispiel 4 a) beschrieben, werden 1 g 9α-Chlor, 16α-methyl-prednisolon-17-äthylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *9α-Chlor, 16α-methyl-prednisolon-17-äthylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g 9α-Chlor, 16α-methyl-prednisolon-17-äthylcarbonat mit Methansulfonsäurechlorid umgesetzt und aufgearbeitet. Man erhält nach dem Kristallisieren aus Äther das 9α-Chlor, 16α-methyl-prednisolon-17-äthyl-carbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende 6α-Chlor, 16α-methyl-prednisolon-17-äthylcarbonat-21-p-toluolsulfonat bzw. -21-p-chlorbenzolsulfonat.

c) Das für die Reaktion zunächst benötigte 6α-Chlor, 16α-methyl-di-äthyl-orthocarbonat ($R_F \cong 0,6$) wird gemäß DBP 1 668 079 aus 6α-Chlor, 16α-methyl-prednisolon und Tetra-äthyl-orthocarbonat hergestellt.

Anschließend wird ersteres in gleicher Weise, wie in Beispiel 1 c) beschrieben, zu 6α-Chlor, 16α-methyl-prednisolon-17-äthylcarbonat ($R_F \cong 0,4$) hydrolisiert.

Beispiel 40

a) In gleicher Weise, wie in Beispiel 4 a) beschrieben, werden 1 g 9$\alpha$-Chlor-prednisolon-17-äthylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *9$\alpha$-Chlor-prednisolon-17-äthylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g 9$\alpha$-Chlor-prednisolon-17-äthylcarbonat mit Methansulfonsäurechlorid umgesetzt und aufgearbeitet. Man erhält nach dem Kristallisieren aus Äther das 9$\alpha$-Chlor-prednisolon-17-äthylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende 9$\alpha$-Chlor-prednisolon-17-äthylcarbonat-21-p-toluolsulfonat bzw. -21-p-chlorbenzolsulfonat.

c) Das für die Reaktion zunächst benötigte 9$\alpha$-Chlor-prednisolon-di-äthyl-orthocarbonat ($R_F \cong 0,6$) wird gemäß DBP 1 668 079 aus 9$\alpha$-Chlor-prednisolon und Tetra-äthyl-orthocarbonat hergestellt.

Anschließend wird ersteres in gleicher Weise, wie in Beispiel 1 c) beschrieben, zu 9$\alpha$-Chlor-prednisolon-17-äthylcarbonat ($R_F \cong 0,4$) hydrolisiert.

Beispiel 41

a) In gleicher Weise, wie in Beispiel 4 a) beschrieben, werden 1 g Prednisolon-17-n-propylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *Prednisolon-17-n-propylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,

8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g Prednisolon-17-n-propylcarbonat mit Methansulfonsäurechlorid umgesetzt und aufgearbeitet. Man erhält nach dem Kristallisieren aus Äther das Prednisolon-17-n-propylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende Prednisolon17-n-propylcarbonat-21-p-toluolsulfonat bzw. -21-p-chlorbenzolsulfonat.

c) Das für die Reaktion zunächst benötigte Prednisolon-di-n-propyl-orthocarbonat ($R_F \cong 0,6$) wird gemäß DBP 1 668 079 aus Prednisolon und Tetra-n-propyl-orthocarbonat hergestellt.

Anschließend wird ersteres in gleicher Weise, wie in Beispiel 1 c) beschrieben, zu Prednisolon-17-n-propyl-carbonat ($R_F \cong 0,4$) hydrolisiert.

Beispiel 42

a) In gleicher Weise, wie in Beispiel 4 a) beschrieben, werden 1 g Prednison-17-n-propylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende
1) *Prednison-17-n-propylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g Prednison-17-n-propylcarbonat mit Methansulfonsäurechlorid umgesetzt und aufgearbeitet. Man erhält nach dem Kristallisieren aus Äther das Prednison-17-n-propylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende Prednison-17-n-propylcarbonat-21-p-toluolsulfonat bzw. -21-p-chlorbenzolsulfonat.

c) Das für die Reaktion zunächst benötigte Prednison-di-n-propylorthocarbonat ($R_F \cong 0,6$) wird gemäß DBP 1 668 079 aus Prednison und Tetra-n-propyl-orthocarbonat hergestellt.

Anschließend wird ersteres in gleicher Weise, wie in Beispiel 1 c) beschrieben, zu Prednison-17-n-propyl-carbonat ($R_F \cong 0,4$) hydrolisiert.

Beispiel 43

a) In gleicher Weise, wie in Beispiel 4 a) beschrieben, werden 1 g Cortison-17-n-propylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,

7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende
1) *Cortison-17-n-propylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g Cortison-17-n-propylcarbonat mit Methansulfonsäurechlorid umgesetzt und aufgearbeitet. Man erhält nach dem Kristallisieren aus Äther das Cortison-17-n-propylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende Cortison-17-n-propylcarbonat-21-p-toluolsulfonat bzw. -21-p-chlorbenzolsulfonat.

c) Das für die Reaktion zunächst benötigte Cortison-di-n-propyl-orthocarbonat ($R_F \cong 0,6$) wird gemäß DBP 1 668 079 aus Cortison und Tetra-n-propylorthocarbonat hergestellt.

Anschließend wird ersteres in gleicher Weise, wie in Beispiel 1 c) beschrieben, zu Cortison-17-n-propyl-carbonat ($R_F \cong 0,4$) hydrolisiert.

Beispiel 44

a) In gleicher Weise, wie in Beispiel 4 a) beschrieben, werden 1 g Cortisol-17-n-propylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende
1) *Cortisol-17-n-propylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g Cortisol-17-n-propylcarbonat mit Methansulfonsäurechlorid umgesetzt und aufgearbeitet. Man erhält nach dem Kristallisieren aus Äther das Cortisol-17-n-propylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende Cortisol-17-n-

propylcarbonat-21-p-toluolsulfonat bzw. -21-p-chlorbenzolsulfonat.

c) Das für die Reaktion zunächst benötigte Cortisol-di-n-propylorthocarbonat ($R_F \cong 0{,}6$) wird gemäß DBP 1 668 079 aus Cortisol und Tetra-n-propyl-orthocarbonat hergestellt.

Anschließend wird ersteres in gleicher Weise, wie in Beispiel 1 c) beschrieben, zu Cortisol-17-n-propyl-carbonat ($R_F \cong 0{,}4$) hydrolisiert.

Beispiel 45

a) In gleicher Weise, wie in Beispiel 4 a) beschrieben, werden 1 g Beclomethason-17-n-propylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *Beclomethason-17-n-propylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g Beclomethason-17-n-propylcarbonat mit Methansulfonsäurechlorid umgesetzt und aufgearbeitet. Man erhält nach dem Kristallisieren aus Äther das Beclomethason-17-n-propylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende Beclomethason-17-n-propylcarbonat-21-p-toluolsulfonat bzw. -21-p-chlorbenzolsulfonat.

c) Das für die Reaktion zunächst benötigte Beclomethason-di-n-propyl-orthocarbonat ($R_F \cong 0{,}6$) wird gemäß DBP 1 668 079 aus Beclomethason und Tetra-n-propyl-orthocarbonat hergestellt.

Anschließend wird ersteres in gleicher Weise, wie in Beispiel 1 c) beschrieben, zu Beclomethason-17-n-propyl-carbonat ($R_F \cong 0{,}4$) hydrolisiert.

Beispiel 46

a) In gleicher Weise, wie in Beispiel 4 a) beschrieben, werden 1 g 6$\alpha$-Fluordexamethason-17-n-propylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende
1) *6α-Fluordexamethason-17-n-propylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g 6α-Fluordexamethason-17-n-propylcarbonat mit Methansulfonsäurechlorid umgesetzt und aufgearbeitet. Man erhält nach dem Kristallisieren aus Äther das 6α-Fluordexamethason-17-n-propylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende 6α-Fluordexamethason-17-n-propylcarbonat-21-p-toluolsulfonat bzw. -21-p-chlorbenzolsulfonat.

c) Das für die Reaktion zunächst benötigte 6α-Fluordexamethason-di-n-propyl-orthocarbonat ($R_F \cong 0{,}6$) wird gemäß DBP 1 668 079 aus 6α-Fluordexamethason und Tetra-n-propyl-orthocarbonat hergestellt.

Anschließend wird ersteres in gleicher Weise, wie in Beispiel 1 c) beschrieben, zu 6α-Fluordexamethason-17-n-propyl-carbonat ($R_F \cong 0{,}4$) hydrolisiert.

Beispiel 47

a) In gleicher Weise, wie in Beispiel 4 a) beschrieben, werden 1 g Betamethason-17-n-propylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende
1) *Betamethason-17-n-propylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g Betamethason-17-n-propylcarbonat mit Methansulfonsäurechlorid umgesetzt und aufgearbeitet. Man erhält nach dem Kristallisieren aus Äther das Betamethason-17-n-propylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende Betamethason-17-n-propylcarbonat-21-p-toluolsulfonat bzw. -21-p-chlorbenzolsulfonat.

c) Das für die Reaktion zunächst benötigte Betamethason-di-n-propyl-orthocarbonat ($R_F \cong 0{,}6$) wird gemäß DBP 1 668 079 aus Betamethason und Tetra-n-propyl-orthocarbonat hergestellt.

Anschließend wird ersteres in gleicher Weise, wie in Beispiel 1 c) beschrieben, zu Betamethason-17-n-propyl-carbonat ($R_F \cong 0{,}4$) hydrolisiert.

Beispiel 48

a) In gleicher Weise, wie in Beispiel 4 a) beschrieben, werden 1 g 6α-Fluor-prednisolon-17-n-propylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *6α-Fluor-prednisolon-17-n-propylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g 6α-Fluor-prednisolon-17-n-propylcarbonat mit Methansulfonsäurechlorid umgesetzt und aufgearbeitet. Man erhält nach dem Kristallisieren aus Äther das 6α-Fluorprednisolon-17-n-propylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende 6α-Fluor-prednisolon-17-n-propylcarbonat-21-p-toluolsulfonat bzw. -21-p-chlorbenzolsulfonat.

c) Das für die Reaktion zunächst benötigte 6α-Fluor-prednisolon-di-n-propyl-orthocarbonat ($R_F \cong 0,6$) wird gemäß DBP 1 668 079 aus 6α-Fluor-prednisolon und Tetra-n-propyl-orthocarbonat hergestellt.

Anschließend wird ersteres in gleicher Weise, wie in Beispiel 1 c) beschrieben, zu 6α-Fluor-prednisolon-17-n-propylcarbonat ($R_F \cong 0,4$) hydrolisiert.

Beispiel 49

a) In gleicher Weise, wie in Beispiel 4 a) beschrieben, werden 1 g 16α- oder β-Methyl-prednisolon-17-n-propylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *16α- oder β-Methyl-prednisolon-17-n-propylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,

8) -21-propionat,
9) -21-butyrat,
10) -21-valerat,
11) -21-cyclopropancarbonsäureester,
12) -21-cyclopentylpropionat

b) In gleicher Weise, wie in Beispiel 2 f) beschrieben, werden 3 g 16α- oder β-Methyl-prednisolon-17-n-propylcarbonat mit Methansulfonsäurechlorid umgesetzt und aufgearbeitet. Man erhält nach dem Kristallisieren aus Äther das 16α- oder β-Methyl-prednisolon-17-n-propylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende 16α- oder β-Methyl-prednisolon-17-n-propylcarbonat-21-p-toluolsulfonat bzw. -21-chlorbenzolsulfonat.

c) Das für die Reaktion zunächst benötigte 16α- oder β-Methylprednisolon-di-n-propyl-orthocarbonat ($R_F$≅0.6) wird, gemäß DBP 1 668 079 aus 16α- oder β-Methyl-prednisolon und Tetra-n-propyl-orthocarbonat hergestellt.

Anschließend wird ersteres in gleicher Weise, wie in Beispiel 1 c) beschrieben, zu 16α- oder β-Methyl-prednisolon-17-n-propyl-carbonat ($R_F$≅0,4) hydrolisiert.

Beispiel 50

a) in gleicher Weise, wie in Beispiel 4 a) beschrieben, werden 1 g 6α,16α- oder β-Dimethyl-prednisolon-17-n-propylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *6α,16α- oder β-Dimethyl-prednisolon-17-n-propylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g 6α, 16α- oder β-Dimethyl-prednisolon-17-n-propylcarbonat mit Methansulfonsäurechlorid umgesetzt und aufgearbeitet. Man erhält nach dem Kristallisieren aus Äther das 6α, 16α- oder β-Dimethyl-prednisolon-17-n-propylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende 6α, 16α- oder β-Dimethyl-prednisolon-17-n-propylcarbonat-21-p-toluolsulfonat bzw. -21-p-chlorbenzolsulfonat.

c) Das für die Reaktion zunächst benötigte 6α, 16α oder β-Dimethyl-prednisolon-di-n-propyl-orthocarbonat ($R_F$≅0,6) wird gemäß DEP 1 668 079 aus 6α, 16α- oder β-Dimethyl-prednisolon und Tetra-n-propyl-orthocarbonat hergestellt.

Anschließend wird ersteres in gleicher Weise, wie in Beispiel 1 c) beschrieben, zu 6α, 16α- oder β-Dimethyl-prednisolon-17-n-propyl-carbonat ($R_F$≅0,4) hydrolisiert.

Beispiel 51

a) In gleicher Weise, wie in Beispiel 4 a) beschrieben, werden 1 g 9$\alpha$-Chlor, 16$\alpha$-methyl-prednisolon-17-n-propyl-carbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8), 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *9α-Chlor-16α-methyl-prednisolon-17-n-propyl-carbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-propionat,
8) -21-propionate,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g 9$\alpha$-Chlor,16$\alpha$-methyl-prednisolon-17-n-propyl-carbonat mit Methansulfonsäurechlorid umgesetzt und aufgearbeitet. Man erhält nach dem Kristallisieren aus Äther das 9$\alpha$-Chlor,16$\alpha$-Methyl-prednisolon-17-n-propyl-carbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende 9$\alpha$-Chlor,16$\alpha$-methyl-prednisolon-17-n-propyl-carbonat-21-p-toluolsulfonat bzw. -21-p-chlorbenzolsulfonat.

c) Das für die Reaktion zunächst benötigte 9$\alpha$-Chlor,16$\alpha$-methyl-prednisolon-di-n-propyl-orthocarbonat ($R_F \cong 0{,}6$) wird gemäß DPB 1 668 079 aus 9$\alpha$-Chlor,16$\alpha$-methyl-prednisolon und Tetra-n-propyl-orthocarbonat hergestellt.

Anschließend wird ersteres in gleicher Weise, wie in Beispiel 1 c) beschrieben, zu 9$\alpha$-Chlor,16$\alpha$-methyl-prednisolon-17-n-propyl-carbonat ($R_F \cong 0{,}4$) hydrolisiert.

Beispiel 52

a) In gleicher Weise, wie in Beispiel 4 a) beschrieben, werden 1 g 9$\alpha$-chlor-prednisolon-17-n-propyl-carbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *9α-Chlor-prednisolon-17-n-propylcarbonat*-21-methylcarbonat
2) -21-äthylcarbonat,
3) -21-n-propylcarbonat,
4) -21-n-butylcarbonat,
5) -21-iso-propylcarbonat,
6) -21-iso-butylcarbonat,

7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g 9α-Chlor-prednisolon-17-n-propyl-carbonat mit Methansulfonsäurechlorid umgesetzt und aufgearbeitet. Man erhält nach dem Kristallisieren aus Äther das 9'α-Chlor-prednisolon-17-n-propylcarbonat-21-methansulfonat.

Wird anstatt des Methansulfonsäurechlorids ein aquimolare Menge des p-Toluolsulfonsäure- oder des p-Chlorbenzolsulfonsäurechlorids eingesetzt, so erhält man das entsprechende 9α-Chlor-prednisolon-17-n-propyl-carbonat-21-p-toluolsulfonat bzw. -21-p-chlorbenzolsulfonat.

c) Das für die Reaktion zunächst benötigte 9α-Chlor-Prednisolon-di-n-propyl-orthocarbonat ($R_F \cong 0,6$) wird gemäß DBP 1 668 079 aus 9α-Chlor-prednisolon und Tetra-n-propylorthocarbonat hergestellt.

Anschließend wird ersteres in gleicher Weise, wie in Beispiel 1 c) beschrieben, zu 9α-Chlor-prednisolon-17-n-propyl-carbonat ($R_F \cong 0,4$) hydrolisiert.

Beispiel 53

a) In gleicher Weise, wie in Beispiel 4 a) beschrieben, werden 1 g Prednisolon-17-n-butylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *Prednisolon-17-n-propyl-carbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g Prednisolon-17-n-butylcarbonat mit Methansulfonsäurechlorid umgesetzt und aufgearbeitet. Man erhält nach dem Kristallisieren aus Äther das Prednisolon-17-n-butylcarbonat-21-methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende Prednisolon-17-n-butylcarbonat-21-p-toluolsulfonat bzw. -21-p-chlorbenzolsulfonat.

c) das für die Reaktion zunächst benötigte Prednisolon-di-(n-butyl)-orthocarbonat ($R_F=0,6$) wird gemäß DBP 1 668 079 aus Prednisolon und Tetra-n-butyl-orthocarbonat hergestellt. Anschließend wird ersteres in gleicher Weise, wie in Beispiel 1 c) beschrieben, zu Prednisolon-17-n-butyl-carbonat ($R_F=0,4$) hydrolisiert.

Beispiel 54

a) In gleicher Weise, wie in Beispiel 4 a) beschrieben, werden 1 g Prednison-17-n-butylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,

5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende.
1) *Prednison-17-n-butylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g Prednison-17-n-butylcarbonat mit Methansulfonsäurechlorid umgesetzt und aufgearbeitet. Man erhält nach dem Kristallisieren aus Äther das Prednison-17-n-butylcarbonat-21-methansulfonat.

Wird anstatt des Methansulfonsäurechlorids eine aquimolare Menge des p-Toluolsulfonsäure- oder des p-Chlorbenzolsulfonsäurechlorids eingesetzt, so erhält man des entsprechende Prednison-17-n-butylcarbonat-21-p-toluolsulfonat bzw. -21-p-chlorbenzosulfonat.

c) Das für die Reaktion zunächst benötigte Prednison-di-(n-butyl)-orthocarbonat ($R_F$=0,6) wird gemäß DBP 1 668 079 aus Prednison und Tetra-n-butyl-orthocarbonat hergestellt. Anschließend wird ersteres in gleicher Weise, wie in Beispiel 1 c) beschrieben, zu Prednison-17-n-butyl-carbonat ($R_F$=0,4) hydrolisiert.

Beispiel 55

a) In gleicher Weise, wie in Beispiel 4 a) beschrieben, werden 1 g Cortison-17-n-butylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende
1) *Cortison-17-n-butylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g Cortison-17-n-butylcarbonat mit Methansulfonsäurechlorid umgesetzt und aufgearbeitet. Man erhält nach dem Kristallisieren aus Äther das Cortison-17-n-butylcarbonat-21-methansulfonat.

Wird anstatt des Methansulfonsäurechlorids eine aquimolare Menge des p-Toluolsulfonsäure oder des p-Chlorbenzolsulfonsäurechlorids eingesetzt, so erhält man das entsprechende Cortison-17-n-butylcarbonat-21-p-toluolsulfonat bzw. -21-p-chlorobenzolsulfonat.

c) Das für die Reaktion zunächst benötigte Cortison-di-(n-butyl)-orthocarbonat ($R_F$=0,6) wird gemäß DBP 1 668 079 aus Cortison und Tetra-n-butyl-orthocarbonat hergestellt.

Anschließend wird ersteres in gleicher Weise, wie in Beispiel 1 c) beschrieben, zu Cortison-17-n-butyl-carbonat ($R_F$=0,4) hydrolisiert.

Beispiel 56

a) In gleicher Weise, wie in Beispiel 4 a) beschrieben, werden 1 g Cortisol-17-n-butylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäuremethylesters umgesetzt und aufgearbeitet.

Man erhält jeweils das entsprechende

1) *Cortisol-17-n-butylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g Cortisol-17-n-butylcarbonat mit Methansulfonsäurechlorid umgesetzt und aufgearbeitet. Man erhält nach dem Kristallisieren aus Äther das Cortisol-17-n-butylcarbonat-21-methansulfonat.

Wird anstatt des Methansulfonsäurechlorids eine aquimolare Menge des p-Toluolsulfonsäure- oder des p-Chlorbenzolsulfonsäurechlorids eingesetzt, so erhält man das entsprechende Cortisol-17-n-butylcarbonat-21-p-toluolsulfonat bzw. -21-p-chlorbenzolsulfonat.

c) Das für die Reaktion zunächst benötigte Cortisol-di(n-butyl)-orthocarbonat ($R_F$=0,6) wird gemäß DBP 1 668 079 aus Cortisol und Tetra-n-butyl-orthocarbonat hergestellt. Anschließend wird ersteres in gleicher Weise, wie in Beispiel 1 c) beschrieben, zu Cortisol-17-n-butyl-carbonat ($R_F$=0,4) hydrolisiert.

Beispiel 57

a) In gleicher Weise, wie in Beispiel 4 a) beschrieben, werden 1 g Beclomethason-17-n-butylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *Beclomethason-17-n-butylcarbonat*-21-methylcarbonat,
2) -21-äthylcarbonat,
3) -21-n-propylcarbonat,
4) -21-n-propylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g Beclomethason-17-n-butylcarbonat mit Methansulfonsäurechlorid umgesetzt und aufgearbeitet. Man erhält nach dem Kristallisieren aus Äther das Beclomethason-17-n-butylcarbonat-21-methansulfonat.

Wird anstatt des Methansulfonsäurechlorids eine aquimolare Menge des p-Toluolsulfonsäure- oder des p-Chlorbenzolsulfonsäurechlorids eingesetzt, so erhält man das entsprechende Beclomethason-17-n-butylcarbonat-21-p-toluolsulfonat bzw. -21-p-chlorbenzolsulfonat.

c) Das für die Reaktion zunächst benötigte Beclomethason-di-(n-butyl)-orthocarbonat ($R_F$=0,6) wird gemäß DBP 1 668 079 aus Beclomethason und Tetra-n-butyl-orthocarbonat hergestellt.

Anschließend wird ersteres in gleicher Weise, wie in Beispiel 1 c) beschrieben, zu Beclomethason-17-n-butylcarbonat ($R_F$=0,4) hydrolisiert.

Beispiel 58

a) In gleicher Weise, wie in Beispiel 4 a) beschrieben, werden 1 g 6α-Fluordexamethason-17-n-butylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *6α-Fluordexamethason-17-n-butylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Biespiel 2 f) beschrieben, werden 3 g 6α-Fluordexamethason-17-n-butylcarbonat mit Methansufonsäurechlorid umgesetzt und aufgearbeitet. Man erhält nach dem Kristallisieren aus Äther das 6α-Fluordexamethason-17-n-butylcarbonat-21-methansulfonat.

Wird anstatt des Methansulfonsäurechlorids eine aquimolare Menge des p-Toluolsulfonsäure- oder des p-Chlorbenzolsulfonsäurechlorids eingesetzt, so erhält man das entsprechende 6α-Fluordexamethason-17-n-butylcarbonat-21-p-toluolsulfonat bzw. -21-p-chlorbenzosulfonat.

c) Das für die Reaktion zunächst benötigte 6α-Fluordexamethason-di-(n-butyl)-orthocarbonat ($R_F$=0,6) wird gemäß DBP 1 668 079 aus 6α-Fluordexamethason und Tetra-n-butylorthocarbonat hergestellt.

Anschließend wird ersteres in gleicher Weise, wie in Beispiel 1 c) beschrieben, zu 6α-Fluordexamethason-17-n-butylcarbonat ($R_F$=0,4) hydrolisiert.

Beispiel 59

a) In gleicher Weise, wie in Beispiel 4 a) beschrieben, werden 1 g Betamethason-17-n-butylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *Betamethason-17-n-butylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g Betamethason-17-n-butylcarbonat mit Methansulfonsäurechlorid umgesetzt und aufgearbeitet. Man erhält nach dem Kristallisieren aus Äther das Betamethason-17-n-butylcarbonat-21-methansulfonat.

Wird anstatt des Methansulfonsäurechlorids eine aquimolare Menge des p-Toluolsulfonsäure- oder des p-Chlorbenzolsulfonsäurechlorids eingesetzt, so erhält man das entsprechende Betamethason-17-n-butylcarbonat-21-p-toluolsulfonat bzw. -21-p-chlorbenzosulfonat.

c) Las für die Reaktion zunächst benötigte Betamethason-di-(n-butyl)-orthocarbonat ($R_F$=0,6) wird gemäß DBP 1 668 079 aus Betamethason und Tetra-n-butyl-orthocarbonat hergestellt.

Anschließend wird ersteres in gleicher Weise, wie in Beispiel 1 c) beschrieben, zu Betamethason-17-n-butylcarbonat ($R_F$=0,4) hydrolisiert.

Beispiel 60

a) In gleicher Weise, wie in Biespiel 4a) beschrieben, werden 1 g 6α-Fluorprednisolon-17-n-butylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende.

1) *6α-Fluorprednisolon-17-n-butylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,

9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g 6α-Fluorprednisolonk-17-n-butylcarbonat mit Methansulfonsäurechlorid umgesetzt und aufgearbeitet. Man erhält nach dem Kristallisieren aus Äther das 6α-Fluorprednisolon-17-n-butylcarbonat-21-methansulfonat.

Wird anstatt des Methansulfonsäurechlorids eine aquimolare Menge des p-Toluolsulfonsäure- oder des p-Chlorbenzolsulfonsäurechlorids eingesetzt, so erhält man das entsprechende 6α-Fluorprednisolon-17-n-butylcarbonat-21-p-toluolsulfonat bzw. -21-p-chlorbenzolsulfonat.

c) Das für die Reaktion zunächst benötigte 6α-Fluorprednisolon-di-(n-butyl)-orthocarbonat ($R_F$=0,6) wird gemäß DBP 1 668 079 aus 6α-Fluorprednisolon und Tetra-n-butylorthocarbonat hergestellt.

Anschließend wird esteres in gleicher Weise, wie in Beispiel 1 c) beschrieben, zu 6α-Fluorprednisolon-17-n-butyl-carbonat ($R_F$=0,4) hydrolysiert.

Beispiel 61

a) In gleicher Weise, die in Beispiel 4 a) beschrieben, werden 1 g 16α- oder β-Methyl-prednisolon-17-n-butylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8), 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *16α- oder β-Methyl-prednisolon-17-n-butylcarbonat-*21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat,

b) In gleicher Weise, wie in Beispiel 2 f) beschrieben, werden 3 g 16α- oder β-Methyl-prednisolon-17-n-butylcarbonat mit Methansulfonsäurechlorid umgesetzt und aufgearbeitet. Man erhält nach dem Kristallisieren aus Äther das 16α- oder β-Methyl-prednisolon-17-n-butylcarbonat-21-methansulfonat.

Wird anstatt des Methansulfonsäurechlorid eine aquimolare Menge des p-Toluolsulfonsäure oder des p-Chlorbenzolsulfonsäurechlorids eingesetzt, so erhält man das entsprechende 16α- oder β-Methyl-prednisolon-17-n-butylcarbonat-21-p-toluolsulfonat bzw. -21-p-chlorbenzolsulfonat.

c) Das für die Reaktion zunächst benötigte 16α- oder β-Methylprednisolon-di-(n-butyl)-orthocarbonat ($R_F$=0,6) wird gemäß DBP 1 668 079 aus 16α- oder β-Methyl-prednisolon und Tetra-n-butyl-orthocarbonat hergestellt.

Anschließend wird ersteres in gleicher Weise, wie in Beispiel 1 c) beschrieben, zu 16α- oder β-Methyl-prednisolon-17-n-butyl-carbonat ($R_F$=0,4) hydrolisiert.

Beispiel 62

a) In gleicher Weise, wie in Beispiel 4 a) beschrieben, werden 1 g 6α,16α- oder β-Dimethyl-prednisolon-17-n-butylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,

5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid satt des Chlorameisensäure-methylesters umgesetzt und aufgearbeitet.

Man erhält jeweils das entsprechende
1) *6α,16α-oder β-Dimethyl-prednisolon-17-n-butylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g 6α,16α- oder β-Dimethyl-prednisolon-17-n-butylcarbonat mit Methansulfonsäurechlorid umgestetzt und aufgearbeitet. Man erhalt nach dem Kristallisieren aus Äther das 6α,16α- oder β-Dimethyl-prednisolon-17-n-butylcarbonat-21-methansulfonat.

Wird anstatt des Methansulfonsäurechlorids eine aquimolare Menge des p-Toluolsulfonsäure- oder des p-Chlorbenzolsulfonsäurechlorids eingesetzt, so erhält man das entsprechende 6α,16α- oder β-Dimethyl-prednisolon-17-n-butylcarbonat-21-p-toluolsulfonat bzw. -21-p-chlorbenzolsulfonat.

c) Das für die Reaktion zunächst benötigte 6α,16α- oder β-Dimethyl-prednisolon-di-(n-butyl)-orthocarbonat ($R_F$=0,6) wird gemäß DBP 1 668 079 aus 6α,16α- oder β-Dimethyl-prednisolon und Tetra-n-butyl-orthocarbonat hergestellt.

Anschließend wird ersteres in gleicher Weise, wie in Beispiel 1 c) beschrieben, zu 6α,16α- oder β-Dimethyl-prednisolon-17-n-butyl-carbonat ($R_F$=0,4) hydrolisiert.

Beispiel 63

a) In gleicher Weise, wie in Beispiel 4 a) beschrieben, werden 1 g 9α-Chlor,16α-methyl-prednisolon-17-n-butylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12), 1,3 ml Cyclopentylpropionsäurechloride statt des Chlorameisensäure-methylesters umgesetzt und aufgearbeitet.

Man erhält jeweils das entsprechende
1) *9α-Chlor,16α-methyl-prednisolon-17-n-butylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g 9α-Chlor,16α-methyl-

prednisolon-17-n-butylcarbonat mit Methansulfonsäurechlorid umgesetzt und aufgearbeitet. Man erhält nach dem Kristallisieren aus Äther das 9α-Chlor,16α-Methyl-Prednisolon-17-n-butylcarbonat-21-methansulfonat.

Wird anstatt des Methansulfonsäurechlorids eine aquimolare Menge des p-Toluolsulfonsäure- oder des p-Chlorbenzolsulfonsäurechlorids eingesetzt, so erhält man das entsprechende 6α-Chlor,16α-methyl-prednisolon-17-n-butylcarbonat-21-p-toluolsulfonat bzw. -21-p-chlorbenzolsulfonat.

c) Das für die Reaktion zunächst benötigte 6α-Chlor,16α-methyl-prednisolon-di-(n-butyl)-orthocarbonat ($R_F$=0,6) wird gemäß DBP 1 668 079 aus 6α-Chlor,16α-methyl-prednisolon und Tetra-n-butyl-orthocarbonat hergestellt.

Anschließend wird ersteres in gleicher Weise, wie in Beispiel 1 c) beschrieben, zu 6α-Chlor,16α-Methyl-prednisolon-17-n-butyl-carbonat ($R_F$=0,4) hydrolisiert.

Beispiel 64

a) In gleicher Weise, wie in Beispiel 4 a) beschrieben, werden 1 g 9α-Chlor-prednisolon-17-n-butylcarbonat mit

1) 0.8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9), 0,9 Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende

1) *9α-Chlor-prednisolon-17-n-butylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g 9α-Chlor-prednisolon-17-n-butylcarbonat mit Methansulfonsäurechlorid umgesetzt und aufgearbeitet. Man erhält nach dem Kristallisieren aus Äther das 9α-Chlor-Prednisolon-17-n-butylcarbonat-21-methansulfonat.

Wird anstatt des Methansulfonsäurechlorids eine aquimolare Menge des p-Toluolsulfonsäure- oder des p-Chlorbenzolsulfonsäurechlorids eingesetzt, so erhält man das entsprechende 9α-Chlor-prednisolon-17-n-butylcarbonat-21-p-toluolsulfonat bzw. -21-p-chlorbenzolsulfonat.

c) Das für die Reaktion zunächst benötigte 9α-Chlor-prednisolon di-(n-butyl)-orthocarbonat ($R_F$=0,6) wird gemäß DBP 1 668 079 aus 9α-Chlor-prednisolon und Tetra-n-butyl-orthocarbonat hergestellt. Anschließend wird ersteres in gleicher Weise, wie in Beispiel 1 c) beschrieben, zu 9α-Chlor-prednisolon-17-n-butylcarbonat ($R_F$=0,4) hydrolisiert.

Beispiel 65

a) In gleicher Weise, wie in Beispiel 4 a) beschrieben, werden 1 g Prednisolon-17-valerylcarbonat mit

1) 0.8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,

11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende
1) *Prednisolon-17-valerylcarbonat*-21-methylcarbonat
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g Prednisolon-17-valerylcarbonat mit Methansulfonsäurechlorid umgesetzt und aufgearbeitet. Man erhält nach dem Kristallisieren aus Äther das Prednisolon-17-valerylcarbonat-21-methansulfonat.

Wird anstatt des Methansulfonsäurechlorids eine aquimolare Menges des p-Toluolsulfonsäure- oder des p-Chlorobenzolsulfonsäurechlorids eingesetzt, so erhält man das entsprechende Prednisolon-17-valerylcarbonat-21-p-toluolsu fonat bzw. -21-p-chlorbenzolsulfonat.

c) Das für die Reaktion zunächst benötigte Prednisolon-divaleryl-orthocarbonat ($R_F \cong 0,6$) wird gemäß DBP 1 668 0 9 aus Prednisolon und Tetra-valeryl-orthocarbonat hergestellt. Anschließend wird ersteres in gleicher Weise, wie in Beispiel 1 c) beschrieben, zu Prednisolon-17-valerylcarbonat ($R_F \cong 0,4$) hydrolisiert.

## Beispiel 66

a) In gleicher Weise, wie in Beispiel 4 a) beschrieben, werden 1 g Prednisolon-17-valerylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9), 0,9 Buttersäurechlorid,
10) ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende
1) *Prednison-17-valerylcarbonat*-21-methylcarbonat,
2) -21-äthylcarbonat,
3) -21-butylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g Prednison-17-valerylcarbonat mit Methansulfonsäurechlorid umgesetzt und aufgearbeitet. Man erhält nach dem Kristallisiren aus Äther das Prednison-17-valerylcarbonat-21-methansulfonat.

Wird anstatt des Methansulfonsäurechlorids eine aquimolare Menge des p-Toluolsulfonsäure- oder des p-Chlorbenzolsulfonsäurechlorids eingesetzt, so erhält man das entsprechende Prednison-17-valerylcarbonat-21-p-toluolsulfonat bzw. -21-p-chlorbenzolsulfonat.

c) Das für die Reaktion zunächst benötigte Prednison-di-valerylorthocarbonat ($R_F \cong 0,6$) wird gemäß DBP 1 668 079 aus Prednison und Tetra-valeryl-orthocarbonat hergestellt. Anschließend wird

ersteres in gleicher Weise, wie in Beispiel 1 c) beschrieben, zu Prednison-17-valeryl-carbonat ($R_F \cong 0,4$) hydrolisiert.

Beispiel 67

a) In gleicher Weise, wie in Beispiel 4 a) beschrieben, werden 1 g Cortison-17-valerylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende
1) *Cortison-17-valerylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g Cortison-17-valerylcarbonat mit Methansulfonsäurechlorid umgesetzt und aufgearbeitet. Man erhält nach dem Kristallisieren aus Äther das Cortison-17-valerylcarbonat-21-methansulfonat.

Wird anstatt des Methansulfonsäurechlorids eine aquimolare Menge des p-Toluolsulfonsäure- oder des p-Chlorbenzolsulfonsäurechlorids eingesetzt, so erhält man das entsprechende Cortison-17-valeryl-carbonat-21-p-toluolsulfonat bzw. -21-p-chlorbenzolsulfonat.

c) Das für die Reaktion zunächst benötigte Cortison-di-valerylorthocarbonat ($R_F \cong 0,6$) wird gemäß DBP 1 668 079 aus Cortison und Tetra-valeryl-orthocarbonat hergestellt. Anschließend wird ersteres in gleicher Weise, wie in Beispiel 1 c) beschrieben, zu Cortison-17-valeryl-carbonat ($R_F \cong 0,4$) hydrolisiert.

Beispiel 68

a) In gleicher Weise, wie in Beispiel 4 a) beschrieben, werden 1 g Cortisol-17-valerylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet.

Man erhält jeweils das entsprechende
1) *Cortisol-17-valerylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,

7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g Cortisol-17-valerylcarbonat mit Methansulfonsäurechlorid umgesetzt und aufgearbeitet. Man erhält nach dem Kristallisieren aus Äther das Cortisol-17-valerylcarbonat-21-methansulfonat.

Wird anstatt des Methansulfonsäurechlorids eine aquimolare Menge des p-Toluolsulfonsäure- oder des p-Chlorbenzolsulfonsäurechlorids eingesetzt, so erhält man des entsprechende Cortisol - 17 - valeryl - carbonat - 21 - p - toluolsulfonat bzw. -21-p-chlorbenzolsulfonat.

c) Das für die Reaktion zunächst benötigte Cortisol-di-valerylorthocarbonat ($R_F \cong 0{,}6$) wird gemäß DBP 1 668 079 aus Cortisol und Tetra-valeryl-orthocarbonat hergestellt. Anschließend wird ersteres in gleicher Weise, wie in Beispiel 1 c) beschrieben, zu Cortisol-17-valeryl-carbonat ($R_F \cong 0{,}4$) hydrolisiert.

Beispiel 69

a) In gleicher Weise, wie in Beispiel 4 a) beschrieben werden g Beclomethason-17-valerylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäuremethylesters umgesetzt und aufgearbeitet. Man erhält jeweils das entsprechende
1) *Beclomethason-17-valerylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g Beclomethason-17-valerylcarbonat mit Methansulfonsäurechlorid umgesetzt und aufgearbeitet. Man erhält nach dem Kristallisieren aus Ather das Beclomethason-17-valerylcarbonat-21-methansulfonat.

Wird anstatt des Methansulfonsäurechlorids eine aquimolare Menge des p-Toluolsulfonsäure- oder des p-Chlorbenzolsulfonsäurechlorids eingesetzt, so erhält man das entsprechende Beclomethason-17-valerylcarbonat-21-p-toluolsulfonat bzw. -21-p-chlorbenzolsulfonat.

c) Das für die Reaktion zunächst benötigte Beclomethason-di-valeryl-orthocarbonat ($R_F \cong 0{,}6$) wird gemäß DBP 1 668 079 aus Beclomethason und Tetra-valeryl-orthocarbonat hergestellt.

Anschließend wird ersteres in gleicher Weise, wie in Beispiel 1 c) beschrieben, zu Beclomethason-17-valeryl-carbonat ($R_F \cong 0{,}4$) hydrolisiert.

Beispiel 70

a) In gleicher Weise, wie in Beispiel 4a) beschrieben, werden 1 g 6α-Fluordexamethason-17-valerylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,

8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet. Man erhält jeweils das entsprechende

1) *6α-Fluordexamethason-17-valerylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g 6α-Fluordexamethason-17-valerylcarbonat mit Methansulfonsäurechlorid umgesetzt und aufgearbeitet. Man erhält nach dem Kristallisieren aus Äther des 6α-Fluordexamethason-17-valerylcarbonat-21-methansulfonat.

Wird anstatt des Methansulfonsäurechlorids eine aquimolare Menge des p-Toluolsulfonsäure- oder des p-Chlorbenzolsulfonsäurechlorids eingesetzt, so erhält man das entsprechende 6α - Fluordexamethason - 17 - valerylcarbonat - 21 - p - toluolsulfonat bzw. -21-p-chlorbenzolsulfonat.

c) Das für die Reaktion zunächst benötigte 6α-Fluordexamethason-di-valeryl-orthocarbonat ($R_F \cong 0,6$) wird gemäß DBP 1 668 079 aus 6α-Fluordexamethason und Tetra-valeryl-orthocarbonat hergestellt.

Anschließend wird ersteres in gleicher Weise, wie in Beispiel 1 c) beschrieben, zu 6α-Fluordexamethason-17-valeryl-carbonat ($R_F \cong 0,4$) hydrolisiert.

Beispiel 71

a) In gleicher Weise, wie in Beispiel 4 a) beschrieben, werden 1 g Betamethason-17-valerylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet. Man erhält jeweils das entsprechende

1) *Betamethason-17-valerylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g Betamethason-17-valerylcarbonat mit Methansulfonsäurechlorid umgesetzt und aufgearbeitet. Man erhält nach dem Kristallisieren aus Äther das Betamethason-17-valerylcarbonat-21-methansulfonat.

Wird anstatt des Methansulfonsäurechlorids eine aquimolare Menge des p-Toluolsulfonsäure- oder des p-Chlorbenzolsulfonsäurechlorids eingesetzt, so erhält man das entsprechende Betamethason-17-valerylcarbonat-21-p-toluolsulfonat bzw. -21-p-chlorbenzolsulfonat.

c) Das für die Reaktion zunächst benötigte Betamethason-di-valeryl-orthocarbonat ($R_F \cong 0{,}6$) wird gemäß DBP 1 668 079 aus Betamethason und Tetra-valeryl-orthocarbonat hergestellt. Anschließend wird ersteres in gleicher Weise, wie in Beispiel 1c) beschrieben, zu Betamethason-17-valerylcarbonat ($R_F \cong 0{,}4$) hydrolisiert.

Beispiel 72

a) In gleicher Weise, wie in Beispiel 4 a) beschrieben, werden 1 g 6α-Fluor-prednisolon-17-valerylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet. Man erhält jeweils das entsprechende

1) *6α-Fluor-prednisolon-17-valerylcarbonat-21-methylcarbonat,*
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g 6α-Fluor-prednisolon-17-valerylcarbonat mit Methansulfonsäurechlorid umgesetzt und aufgearbeitet. Man erhält nach dem Kristallisieren aus Äther des 6α-Fluor-prednisolon-17-valerylcarbonat-21-methansulfonat.

Wird anstatt des Methansulfonsäurechlorids eine aquimolare Menge des p-Toluolsulfonsäure- oder des p-Chlorbenzolsulfonsäurechlorids eingesetzt, so erhält man das entsprechende 6α - Fluor - prednisolon - 17 - valerylcarbonat - 21 - p - toluolsulfonat bzw. -21-p-chlorbenzolsulfonat.

c) Das für die Reaktion zunächst benötigte 6α - Fluor - prednisolon - di - valeryl - orthocarbonat ($R_F \cong 0{,}6$) wird gemäß DBP 1 668 079 aus 6α-Fluor-prednisolon und Tetra-valerylorthocarbonat hergestellt.

Anschließend wird ersteres in gleicher Weise, wie in Beispiel 1 c) beschrieben, zu 6α - Fluor - prednisolon - 17 - valeryl - carbonat ($R_F \cong 0{,}4$) hydrolisiert.

Beispiel 73

a) In gleicher Weise, wie in Beispiel 4 a) beschrieben, werden 1 g 16α- oder β - Methyl - prednisolon - 17 - valerylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet. Man erhält jeweils das entsprechende

1) 16α-*oder* β-Methyl-prednisolon-17-valerylcarbonat-21-methylcarbonat,
2) -21-äthylcarbonat,
3) -21-n-propylcarbonat,
4) -21-n-butylcarbonat,

5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g 16α- oder β - Methyl - prednisolon - 17 - valerylcarbonat mit Methansulfonsäurechlorid umgesetzt und aufgearbeitet. Man erhält nach dem Kristallisieren aus Äther das 16α- oder β - Methyl - prednisolon - 17 - valerylcarbonat - 21 - methansulfonat.

Wird anstatt des Methansulfonsäurechlorids eine aquimolare Menge des p-Toluolsulfonsäure- oder des p-Chlorbenzolsulfonsäurechlorids eingesetzt, so erhält man das entsprechende 16α- oder β - Methyl - prednisolon - 17 - valerylcarbonat - 21 - p - toluolsulfonat bzw. -21-p-chlorbenzolsulfonat.

c) Das für die Reaktion zunächst benötigte 16α- oder β - Methyl - prednisolon - di - valeryl - orthocarbonat ($R_F \cong 0,6$) wird gemäß DBP 1 668 079 aus 16α- oder β-Methyl-prednisolon und Tetra-valeryl-orthocarbonat hergestellt.

Anschließend wird ersteres in gleicher Weise, wie in Beispiel 1 c) beschrieben, zu 16α- oder β - Methyl - prednisolon - 17 - valeryl - carbonat ($R_F \cong 0,4$) hydrolisiert.

Beispiel 74

a) In gleicher Weise, wie in Beispiel 4 a) beschrieben, werden 1 g 6α, 16α- oder β-Dimethyl-prednisolon - 17 - valerylcarbonat mit
1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet. Man erhält jeweils das entsprechende
1) *6α,16α- oder β-Dimethyl-prednisolon-17-valerylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g 6α,16α- oder β - Dimethyl - prednisolon - 17 - valerylcarbonat mit Methansulfonsäurechlorid umgesetzt und aufgearbeitet. Man erhält nach dem Kristallisieren aus Äther das 6α, 16α- oder β - Dimethyl - Prednisolon - 17 - valerylcarbonat - 21 - methansulfonat.

Wird anstatt des Methansulfonsäurechlorids eine aquimolare Menge des p-Toluolsulfonsäure- oder des p-Chlorbenzolsulfonsäurechlorids eingesetzt, so erhält man das entsprechende 6α,16α- oder β - Dimethyl - prednisolon - 17 - valerylcarbonat - 21 - p - toluolsulfonat bzw. -21-p-chlorbenzolsulfonat.

c) Das für die Reaktion zunächst benötigte 6α,16α- oder β - Dimethyl - prednisolon - di - valeryl - orthocarbonat ($R_F \cong 0,6$) wird gemäß DBP 1 668 079 aus 6α,16α- oder β-Dimethyl-prednisolon und Tetra-valeryl-orthocarbonat hergestellt.

Anschließend wird ersteres in gleicher Weise, wie in Beispiel 1 c) beschrieben, zu 6α,16α- oder β - Dimethyl - prednisolon - 17 - valeryl - carbonat ($R_F \cong 0,4$) hydrolisiert.

Beispiel 75

a) In gleicher Weise, wie in Beispiel 4 a) beschrieben, werden 1 g 9$\alpha$ - Chlor, 16$\alpha$ - methyl - prednisolon - 17 - valeryl - carbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet. Man erhält jeweils das entsprechende

1) *9$\alpha$-Chlor,16$\alpha$-methyl-prednisolon-17-valerylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g 9$\alpha$ - Chlor,16$\alpha$ - methyl - prednisolon - 17 - valeryl - carbonat mit Methansulfonsäurechlorid umgesetzt und aufgearbeitet. Man erhält nach dem Kristallisieren aus Äther das 9$\alpha$ - Chlor,16$\alpha$ - Methyl - Prednisolon - 17 - valeryl - carbonat - 21 - methansulfonat.

Wird anstatt des Methansulfonsäurechlorids eine aquimolare Menge des p-Toluolsulfonsäure- oder des p-Chlorbenzolsulfonsäurechlorids eingesetzt, so erhält man das entsprechende 6$\alpha$ - Chlor,16$\alpha$ - methyl - prednisolon - 17 - valeryl - carbonat - 21 - p - toluolsulfonat bzw. -21-p-chlorbenzolsulfonat.

c) Das für die Reaktion zunächst benötigte 6$\alpha$ - Chlor,16$\alpha$ - methyl - prednisolon - di - valeryl - orthocarbonat ($R_F \cong 0{,}6$) wird gemäß DBP 1 668 079 aus 6$\alpha$ - Chlor,16$\alpha$ - methyl - prednisolon und Tetra-valeryl-orthocarbonat hergestellt.

Anschließend wird ersteres in gleicher Weise, wie in Beispiel 1 c) beschrieben, zu 6$\alpha$ - Chlor,16$\alpha$ - methyl - prednisolon - 17 - valeryl - carbonat ($R_F \cong 0{,}4$) hydrolisiert.

Beispiel 76

a) In gleicher Weise, wie in Beispiel 4 a) beschrieben, werden 1 g 9$\alpha$-Chlor-prednisolon-17-valerylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclopropancarbonsäurechlorid und
12) 1,3 ml Cyclopentylpropionsäurechlorid statt des Chlorameisensäure-methylesters umgesetzt und aufgearbeitet. Man erhält jeweils das entsprechende

1) *9$\alpha$-Chlor-prednisolon-17-valerylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,

8) -21-propionat,
9) -21-butyrat,
10) -21-valerat,
11) -21-cyclopropancarbonsäureester,
12) -21-cyclopentylpropionat.

b) In gleicher Weise, wie in Beispiel 2 f) beschrieben, werden 3 g 9$\alpha$ - Chlor - prednisolon - 17 - valerylcarbonat mit Methansulfonsäurechlorid umgesetzt und aufgearbeitet. Man erhält nach dem Kristallisieren aus Äther das 9$\alpha$ - Chlor - Prednisolon - 17 - valerylcarbonat - 21 - methansulfonat.

Wird anstatt des Methansulfonsäurechlorids eine aquimolare Menge des p-Toluolsulfonsäure- oder des p-Chlorbenzolsulfonsäurechlorids eingesetzt, so erhält man das entsprechende 9$\alpha$ - Chlor - prednisolon - 17 - valerylcarbonat - 21 - p - toluolsulfonat bzw, -21-p-chlorbenzolsulfonat.

c) Das für die Reaktion zunächst benötigte 9$\alpha$ - Chlor - prednisolon - di - valeryl - orthocarbonat ($R_F \cong 0{,}6$) wird gemäß DBP 1,668 079 aus 9$\alpha$-Chlor-prednisolon und Tetra-valerylorthocarbonat hergestellt.

Anschließend wird ersteres in gleicher Weise, wie in Beispiel 1 c) beschrieben, zu 9$\alpha$ - Chlor - prednisolon - 17 - valeryl - carbonat ($R_F \cong 04$) hydrolisiert.

Beispiel 77

6 g 6, 16$\alpha$-Dimethyl-4,6-pregnadien-11$\beta$-17$\alpha$,21-triol-3,2-c-2-phenylpyrazol (= Bimedrazol, diese Abkürzung wird auch im folgenden für diese vollständige Nomeklatur benutzt) werden in 125 g abs. Dioxan gelöst und nacheinander mit 500 mg p-Toluolsulfonsäure und 16,5 ml Tetraäthylorthocarbonat versetzt. Nach 6 Stunden Rühren bei 20°C fügt man zur Neutralisation der Säure einige Tropfen Pyridin hinzu, gießt in Wasser ein, wobei ein Öl ausfällt, das über ein Faltenfilter abfiltriert wird. Man nimmt das Öl mit Methylenchlorid auf, wäscht den Extrakt mit Wasser, trocknet und destilliert im Vakuum bis zur Trockne. Man erhält 7,9 g Bimedrazol-17,21-diäthylcarbonat als Schaum.

IR (KBr):
3560, 3400, 2980, 2930, 2880, 1720, 1595, 1500, 1200, 1130, 1035, 755 $cm^{-1}$
UV ($CH_3OH$):
$\lambda_{max\ 1} = 213\ m\mu\ (\varepsilon = 20600)$
$\lambda_{max\ 1} = 280\ m\mu\ (\varepsilon = 17100)$
DC: (Laufmittel: Methylenchlorid/Methanol = 19:1,1 entwickelt):
$R_f = 0{,}45$ (Reaktionsprodukt)
$R_f = 0{,}10$ (Ausgangsprodukt Bimedrazol)

Beispiel 78

a) Eine Lösung von 3 g Bimedrazol-17,21-diäthylorthocarbonat in 120 ml Eisessig und 0,6 ml Wasser wird 5 Stunden bei 22°C stehen gelassen. Eine DC-Überprüfung ergab, daß nach dieser Zeit eine optimale Menge an dem gewünschten Bimedrazol-17-äthylcarbonat vorhanden war. Man gießt das Reaktionsgemisch in 1,5 ml Wasser ein, das mit Ammoniak-Lösung auf pH = 5 gebracht worden war, wobei ein amorpher Niederschlag ausfällt. Nach dem Abfiltrieren, Waschen mit Wasser und Trocknen erhält man nach dem Dispergieren 2,8 g amorphes Bimedrazol-17-äthyl-carbonat.

IR:
3420—3500 (breit), 2940, 2880, 1735, 1715, 1595, 1500, 1265, 760 $cm^{-1}$
DC:
$R_f = 0{,}25$
UV:
$\lambda_{max1} = 312\ m\mu\ (= 20600)$
$\lambda_{max2} = 280\ m_7u\ (= 17100)$

Beispiel 79

a) In gleicher Weise wie in Beispiel 4 a) beschrieben, werden 1 g Bimedrazol-17-äthylcarbonat mit

1) 0,8 ml Chlorameisensäuremethylester,
2) 0,8 ml Chlorameisensäureäthylester,
3) 0,9 ml Chlorameisensäure-n-propylester,
4) 0,9 ml Chlorameisensäure-n-butylester,
5) 1,0 ml Chlorameisensäure-iso-propylester,
6) 1,0 ml Chlorameisensäure-iso-butylester,
7) 0,8 ml Acetylchlorid,
8) 0,8 ml Propionsäurechlorid,
9) 0,9 ml Buttersäurechlorid,
10) 1 ml Valeriansäurechlorid,
11) 1 ml Cyclocarbonsäurechlorid und

12) 1,3 ml Cyclopentylpropionsäurechlorid statt des Chlorameisensäure-methylesters umgesetzt und aufgearbeitet. Man erhält jeweils das entsprechende

1) *Bimedrazol-17-äthylcarbonat*-21-methylcarbonat,
2) -21-äthylcarbonat,
3) -21-n-propylcarbonat,
4) -21-n-butylcarbonat,
5) -21-iso-propylcarbonat,
6) -21-iso-butylcarbonat,
7) -21-acetat,
8) -21-propionat,
9) -21-butyrat,
10) -21-valerat,
11) -21-cyclopropancarbonsäureester,
12) -21-cyclopentylpropionat.

b) in gleicher Weise, wie in Beispiel 2 f) beschrieben, werden 3 g Bimedrazol-17-äthylcarbonat mit Methansulfonsäurechlorid umgesetzt und aufgearbeitet. Man erhält nach dem Kristallisieren aus Äther das Bimedrazol - 17 - äthylcarbonat - 21 - methansulfonat.

Wird anstatt des Methansulfonsäurechlorids eine äquimolare Menge des p-Toluolsulfonsäure- oder des p-Chlorbenzolsulfonsäurechlorids eingesetzt, so erhält man das entsprechende Bimedrazol - 17 - äthylcarbonat - 21 - p - toluolsulfonat bzw. -21-p-chlorbenzolsulfonat.

Beispiel 80

In gleicher Weise, wie oben beschrieben, werden aus Bimedrazol-17-n-propylcarbonat (Darstellung: Bimedrazol + Tetra-n-propyl-orthocarbonat statt Tetraäthylorthocarbonat ergibt zunächst das amorphe Bimedrazol - 17,21 - di - n - propylorthocarbonat, das dann analog in Eisessig/Wasser selektiv solvolysiert wird) die entsprechenden -21-carbonsäureester, -21-carbonate sowie -21-sulfonsäureester, dargestellt.

Beispiel 81

Zu einer Lösung von 1 g Dexamethason-17-äthylcarbonat in 30 ml abs. Tetrahydrofuran und 8 ml abs. Pyridin gibt man bei 20°C 1 ml Chloressigsäureanhydrid. Nach etwa 3 Tagen Rühren bei Raumtemperatur gießt man das Reaktionsgemisch in Kochsalz enthaltendes Wasser ein, extrahiert mit Methylenchlorid, wäscht mit Wasser, trocknet und engt im Vakuum ein, Der erhaltene Schaum liefert nach Umkristallisation aus Aceton/Äther das Dexamethason-17-äthylcarbonat-21-chloracetat vom Schmp. 209°C:

IR: 3460, 1730, 1660, 1615, 1600, 1260 $cm^{-1}$
MS: $M^+ = 540$

Werden anstelle des Dexamethason-17-äthylcarbonats das Cortisol-, Cortison-, Prednisolon, Prednison-, 6α-Methylprednisolon-, 6α-Fluor-prednisolon-, Betamethason-, Beclomethason-, 9α-Chlor, 16α-methyl-prednisolon-, 9α- Fluor - dexamethason - 17 - äthylcarbonat in die Reaktion eingesetzt, so erhält man nach analoger Reaktionsführung und Aufarbeitung die entsprechenden 21-Chloracetate der oben angeführten Corticoid-17-äthylcarbonate.

Werden anstelle des Dexamethason- und der anderen oben angegebenen Corticoid-17-äthylcarbonate die homologen Corticoid-17-n-propylöcarbonate in die Reaktion eingesetzt, so erhält man nach analoger Reaktionsführung und Aufarbeitung die entsprechenden Corticoid - 17 - n - propylcarbonat - 21 - chloracetate.

Beispiel 82

Zu einer Lösung von 2,5 g Dexamethason-17,21-bis-[äthylcarbonat] (hergestellt nach Beispiel 2) in 75 ml Aceton P.A. werden bei 0°C und unter Rühren 2 ml einer $CrO_3$-Oxydationslösung (Herstellung: 13,36 g $CrO_3$ werden in 30 ml Wasser gelöst; unter Eiskühlung läßt man dazu 11,5 ml konz. Schwefelsäure zutropfen; anschließend füllt man auf 50 ml auf) zugetropft. Nach 1 Stunde Rühren bei 0°C und 1,5 Stunden Rühren bei 20°C gießt man das Reaktionsgemisch in Wasser ein, das eine zur Neutralisation erforderliche Menge Pyridin oder Alkalicarbonat enthält, extrahiert mehrmals mit Methylenchlorid, wäscht mit Wasser, trocknet und engt im Vakuum ein. Der erhaltene Schaum wird aus Aceton/Diisopropyl-äther umkristallisert und ergibt 2,1 g 11 - Dehy - drodexamethason - 17,21 - bis - [äthylcarbonat] von Schmp. 212°C.

IR: 1720—1735, 1660, 1625, 1280, 1260 $cm^{-1}$, keine Bande im Bereich um 3420 $cm^{-1}$ (OH) mehr vorhanden !
MS: $M^+ = 533,5$

Werden anstelle des Dexamethosan-17,21-bis-[äthylcarbonat] jeweils die in den vorausgegangen Beispielen dargestellen Corticoid-17-alkylcarbonate, die im 11-Stellung eine Hydroxyl- und in 21-

Stellung entweder eine Alkylcarbonat- oder Alkylcarbonsäureester- oder Alkyl- bzw. Arylsulfonsäureestergruppe aufweisen, in die eben beschriebene Oxydationsreaktion eingesetzt, so erhält man nach analoger Reaktionsführung und Aufarbeitung die entsprechenden 11-Dehydro-corticoid-17-alkylcarbonat *-21-alkylcarbonate,* bzw. *-21-alkylcarbonsäure-* ester, bzw. *-21-alkylsulfonsäureester,* bzw. *-21-aryl-* sulfonsäureester.

**Patentansprüche**

1. Verbindung der Formel I oder II

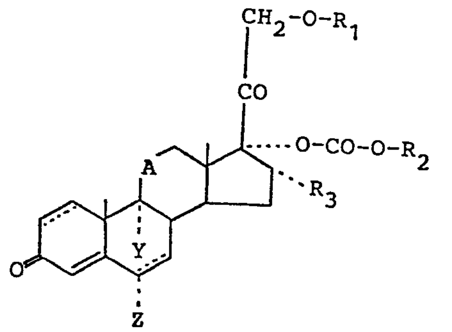

I

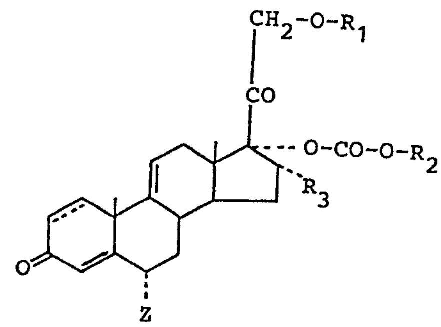

II

in welcher bedeuten:

A $=CHOH$ in beliebiger sterischer Anordnung $=CH_2$ oder $=CO$
Y Wasserstoff, Fluor oder Chlor,
Z Wasserstoff, Fluor, Chlor oder Methyl
$R_3$ Wasserstoff, Fluor, $\alpha$-Methyl, Monofluormethyl oder Difluormethyl,
$R_2$ Alkyl mit 1—8 C-Atomen
$R_1$ Acyl der Formel III

$$—CO—(CH_2)_n—R_4 \qquad III$$

Carbonyloxyalkyl der Formel IV

$$—CO—O—(CH_2)_n—R_4 \qquad IV$$

oder Sulfoalkyl der Formel V

$$—SO_2—R_5 \qquad V$$

in welcher bedeutet
$R_4$ Wasserstoff, Alkyl mit 1—10 C—Atomen, Cycloalkyl mit 3—6 C—Atomen oder, wenn $n \neq 0$, Fluor, Chlor, Brom, Piperidyl
$R_5$ Alkyl mit 1—4 C—Atomen, Phenyl, Methylphenyl, Äthylphenyl, Fluorphenyl, Bromphenyl oder Chlorphenyl
n eine ganze Zahl zwischen 0 und 4.

2. Prednisolon-17-alkylcarbonat-21-carbonsäureester gemäß Anspruch 1.
3. Prednisolon-17-ethylcarbonat-21-propionat.
4. Verfahren zur Herstellung von Verbindungen gemäß Anspruch 1, dadurch gekennzeichnet, daß man Verbindungen der Formeln VI oder VII

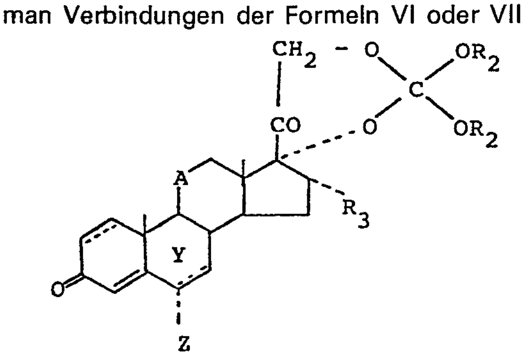

I

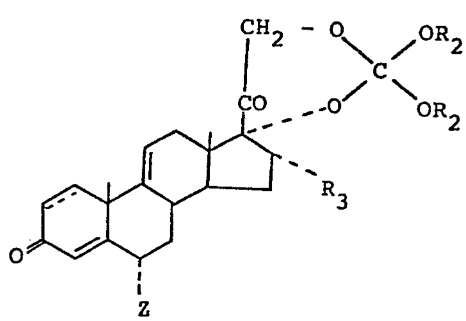

II

mit einer schwachen Säure hydrolysiert und die erhaltene 21-Hydroxy-Verbindung mit einem Halogenid oder Anhydrid einer Carbonsäure der Formel VIII

$$R_4—(CH_2)_n—CO—OH \qquad \text{VIII}$$

einem Halogenformat der Formel IX

$$R_4—(CH_2)_n—O—CO—\text{Halogen} \qquad \text{IX}$$

oder einen Sulfonsäurehalogenid der Formel X

$$R_5—SO_2—\text{Halogen} \qquad \text{X}$$

verestert.

5. Mittel zur Behandlung entzündlicher Dermatosen bestehend aus oder enthaltend eine Verbindung gemäß Anspruch 1, 2 oder 3.

**Revendications**

1. Composés de formule I ou II:

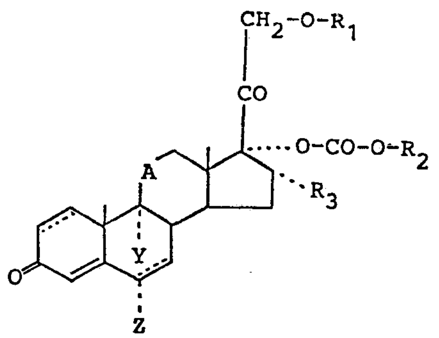

I

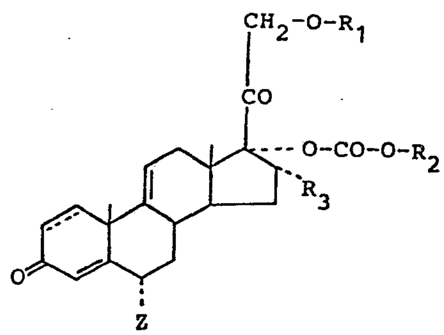

II

dans lesquelles:

A représente =CHOH dans un arrangement stérique quelconque, $=CH_2$ ou =CO,
Y représente l'hydrogène, le fluor ou le chlore,
Z représente l'hydrogène, le fluor, le chlore ou un groupe méthyle,
$R_3$ représente l'hydrogène, le fluor ou un groupe $\alpha$-méthyle, monofluorométhyle ou difluorométhyle,
$R_2$ représente un group alcoyle ayant de 1 à 8 atomes de carbone,
$R_1$ représente un groupe acyle de formule III:

$$—CO—(CH_2)_n—R_4 \qquad \text{III}$$

Carbonyloxyalcoyle de formule IV

$$—CO—O—(CH_2)_n—R_4 \qquad \text{IV}$$

ou sulfoalcoyle de formule V:

$$—SO_2—R_5 \qquad \text{V}$$

dans lesquelles:

$R_4$ représente l'hydrogène, un groupe alcoyle ayant de 1 à 10 atomes de carbone, cycloalcoyle ayant de 3 à 6 atomes de carbone, ou quand n est différent de zéro, le fluor, le chlore, le brome ou un radical pipéridyle,
$R_5$ représente un groupe alcoyle ayant de 1 à 4 atomes de carbone, phényle, méthylphényle, éthylphényle, fluorophényle, bromophényle ou chlorophényle,
n est un nombre entier compris entre 0 et 4.

2. 17-alcoylcarbonate-21-carboxylates de prednisolone selon la revendication 1.

3. 17-éthylcarbonate-21-propionate de prednisolone.

4. Procédé de préparation de composés selon la revendication 1, caractérisé en ce qu'on hydrolyse des composés de formules VI ou VII:

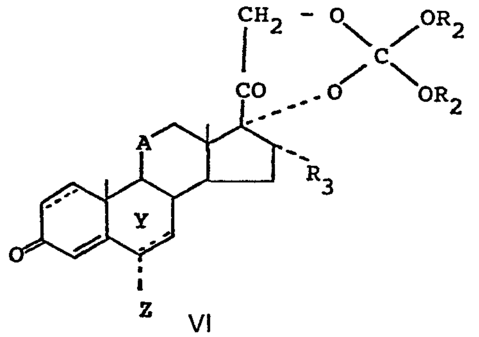

VI

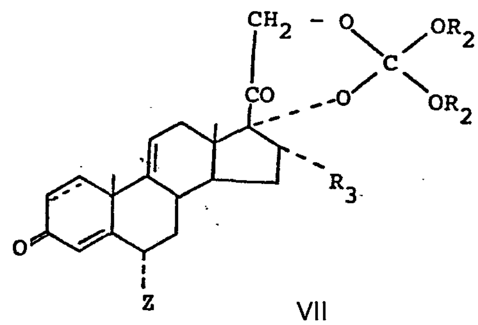

VII

avec un acide faible et on estérifie le composé 21-hydroxy obtenu avec un halogénure ou un anhydride d'un acide carboxylique de formule VIII:

$$R_4—(CH_2)_n—CO—OH \qquad VIII$$

un halogénoformiate de formule IX:

$$R_4—(CH_2)_n—O—CO—\text{halogène}$$

ou un halogénure d'un acide sulfonique de formule X:

$$R_5—SO_2—\text{halogène} \qquad X$$

5. Médicament pour le traitement des dermatoses inflammatoires, constitué de, ou contenant un composé selon l'une quelconque des revendications 1 à 3.

**Claims**

1. Comnpounds of the formulae I or II

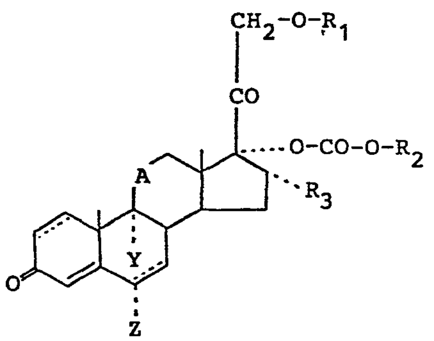

I

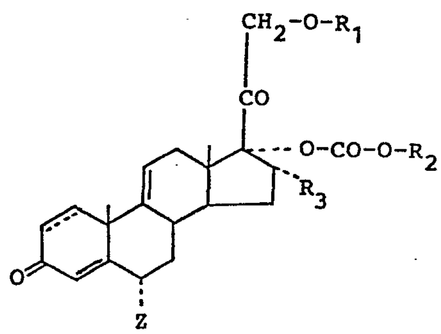

II

in which denotes:

A =CHOH in any desired order $=CH_2$ or =CO
Y hydrogen, fluorine or chlorine,
Z hydrogen, fluorine, chlorine or methyl
$R_3$ hydrogen, fluorine, methyl in $\alpha$-position, monofluoromethyl or difluoromethyl,
$R_2$ alkyl having 1 to 8 C atoms
$R_1$ an acyl radical of the formula III

$$—CO—(CH_2)_n—R_4 \qquad III$$

a carbonyloxyalkyl radical of the formula IV

$$—CO—O—(CH_2)_n—R_4 \qquad IV$$

or a sulfoalkyl radical of the formula V

$$-SO_2-R_5 \qquad V$$

in which

$R_4$ denotes hydrogen, an alkyl radical having 1—10 C atoms or a cycloalkyl radical having 3—6 C atoms or, if $n \neq 0$, $R_4$ represents fluorine, chlorine, bromine or a piperidyl group,

$R_5$ denotes alkyl, having 1—4 C atoms, phenyl, methylphenyl, ethylphenyl, fluorophenyl, bromophenyl or chlorophenyl,

n a number between 0 and 4.

2. Prednisolone-17-alkylcarbonate-21-carboxylic acid ester according to claim 1.

3. Prednisolone-17-ethylcarbonate-21-propionate.

4. Process for the preparation of compounds according to claim 1, which comprises hydrolyzing compounds of the formulae VI or VII

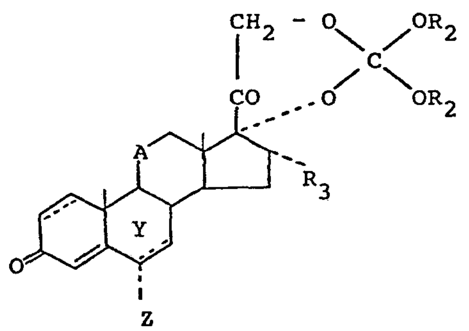

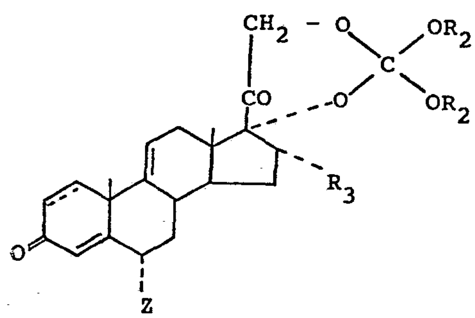

with a weak acid and reacting the 21-hydroxy-compound obtained for forming the esters with a carboxylic acid halide or carboxylic acid anhydride of the formula VIII

$$R_4-(CH_2)_n-CO-OH \qquad VIII$$

or with a halogenoformate of the formula IX

$$R_4-(CH_2)_n-O-CO-halogeno \qquad IX$$

or with a sulfonic acid halide of the formula X

$$R_5-SO_2-halogeno \qquad X$$

5. A composition for treatment of inflammatory dermatosis comprising or containing a compound according to claim 1, 2 or 3.